(19) Europäisches Patentamt
European Patent Office
Office européen des brevets

(11) Publication number : 0 537 937 A2

(12) **EUROPEAN PATENT APPLICATION**

(21) Application number : 92309120.1

(22) Date of filing : 07.10.92

(51) Int. Cl.[5] : C07D 475/02, C07D 475/04, A61K 31/525

(30) Priority : 07.10.91 US 772530

(43) Date of publication of application : 21.04.93 Bulletin 93/16

(84) Designated Contracting States : CH DE FR GB IT LI NL

(71) Applicant : MERCK & CO. INC.
126, East Lincoln Avenue P.O. Box 2000
Rahway New Jersey 07065-0900 (US)

(72) Inventor : Delaszlo, Stephen E.
178 Monmouth Avenue
Atlantic Highlands, NJ 07716 (US)
Inventor : Olson, Richard E.
600 Silverside Road
Wilmington, Delaware 19809 (US)

(74) Representative : Barrett-Major, Julie Diane et al
Merck & Co., Inc. European Patent Department Terlings Park Eastwick Road
Harlow Essex CM20 2QR (GB)

(54) **Substituted pyrazino 2,3-D pyrimidinones as angiotensin II antagonists.**

(57) Novel substituted pyrazino[2,3-d]pyrimidinones of formula (I), are angiotensin II antagonists, useful in the treatment

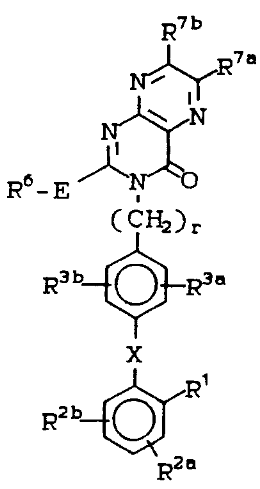

(I)

of hypertension and related disorders and in certain CNS disorders.

EP 0 537 937 A2

SUMMARY OF THE INVENTION

This invention is concerned with novel pyrazino[2,3-d]pyrimidinones of structural formula I

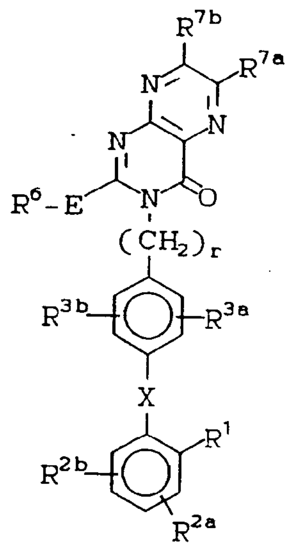

(I)

wherein the substituents are as defined below, which are antagonists of the angiotension II receptors and useful in the treatment of hypertension and disorders related thereto and in the treatment of certain CNS disorders.

This invention is also concerned with pharmaceutical formulations comprising at least one of the novel compounds as active ingredient either alone or in combination with one or more other active ingredients.

The invention is also concerned with methods of treatment of hypertension and related disorders and certain CNS disorders with the novel compounds and the novel pharmaceutical formulations.

The invention is also concerned with novel processes for preparing the novel compounds of this invention.

BACKGROUND OF THE INVENTION

The renin-angiotensin system (RAS) plays a central role in the regulation of blood pressure and is critically involved in the development and maintenance of hypertension as well as congestive heart failure. Angiotensin II (AII), an octapeptide hormone is produced mainly in the blood during the cleavage of angiotensin I by angiotensin converting enzyme (ACE) localized on the endothelium of blood vessels of lung, kidney, and many other organs, and is the end product of the RAS. AII is a powerful arterial vasoconstrictor that exerts its action by interacting with specific receptors present on cell membranes. One of the possible modes of controlling the RAS is angiotensin II receptor antagonism. Several peptide analogs of AII are known to inhibit the effect of this hormone by competitively blocking the receptors, but their experimental and clinical applications have been limited by their partial agonist activity and lack of oral absorption [M. Antonaccio. Clin. Exp. Hypertens. A4, 27-46 (1982); D. H. P. Streeten and G. H. Anderson, Jr. - Handbook of Hypertension, Clinical Pharmacology of Antihypertensive Drugs, ed. A. E. Doyle, Vol. 5, pp. 246-271, Elsevier Science Publisher, Amsterdam, The Netherlands, 1984].

Recently, several non-peptide compounds have been described as AII antagonists. Illustrative of such compounds are those disclosed in U.S. Patents 4,207,324; 4,340,598; 4,576,958; 4,582,847; and 4,880,804; in European Patent Applications 028,834; 245,637; 253,310; 291,969; 323,841; and 324,377; and in articles by A.T. Chiu, et al. [Eur. J. Pharm. Exp. Therap, 157, 13-21 (1988)] and by P.C. Wong, et al. [J. Pharm. Exp. Therap, 247, 1-7(1988), Hypertension, 13, 489-497 (1989)]. All of the U.S. Patents, European Patent Applications 028,834 and 253,310 and the two articles disclose substituted imidazole compounds which are generally bonded through a lower alkyl bridge to a substituted phenyl. Also of interest are European Patent Application 400,974; 411,766 and 419,048 which disclose derivatives of bicyclic compounds substituted with a biphenylalkyl group which are useful as antihypertensive agents.

## DETAILED DESCRIPTION OF THE INVENTION

The novel compounds of this invention have the general formula (I):

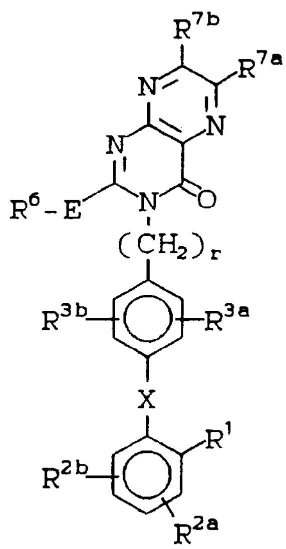

(I)

or a pharmaceutically acceptable salt thereof

wherein

$R^1$ is

(a) $-CO_2R^4$,
(b) $-SO_3R^5$,
(c) $-NHSO_2CF_3$,
(d) $-PO(OR^5)2$,
(e) $-SO_2-NH-R^9$,
(f) $-CONHOR^5$,
(g)

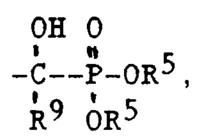

(h)

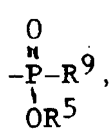

(i) $-SO_2NH$-heteroaryl,
(j) $-CH_2SO_2NH$-heteroaryl,
(k) $-SO_2NH-CO-R^{22}$,
(l) $-CH_2SO_2NH-CO-R^{22}$,
(m) $-CONH-SO_2R^{22}$,
(n) $-CH_2CONH-SO_2R^{22}$,
(o) $-NHSO_2NHCO-R^{22}$,
(p) $-NHCONHSO_2-R^{22}$,

(q)

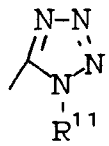 or 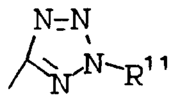,

(r)

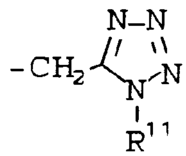 or 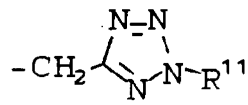,

(s)

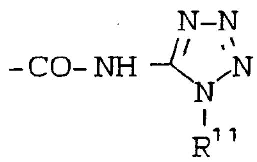 or 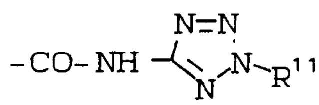,

(t) $-CONHNHSO_2CF_3$ ,
(u) $-SO_2NH-CN$,
(v)

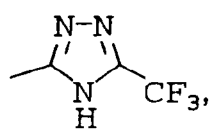,

(w)

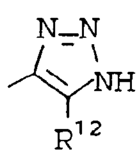,

(x) $-PO(OR^5)(OR^4)$,
(y) $-SO_2NHCONR^4R^{22}$,
$R^{2a}$ and $R^{2b}$ are each independently
(a) H,
(b) halo
(c) $NO_2$,
(d) $NH_2$,
(e) $C_1$-$C_4$-alkylamino,
(f) di($C_1$-$C_4$-alkyl)amino
(g) $SO_2NHR^9$,
(h) $CF_3$,
(i) $C_1$-$C_6$-alkyl,

(j) $C_1$-$C_6$-alkoxy,
(k) $C_1$-$C_6$-alkylthio,
(l) $C_2$-$C_6$-alkenyl,
(m) $C_2$-$C_6$-alkynyl;
(n) aryl,
(o) aryl($C_1$-$C_4$-alkyl), or
(p) $C_3$-$C_7$-cycloalkyl;

$R^{3a}$ is
(a) H,
(b) halo,
(c) $C_1$-$C_6$-alkyl,
(d) $C_1$-$C_6$-alkoxy, or
(e) $C_1$-$C_6$-alkoxy-$C_1$-$C_4$-alkyl;

$R^{3b}$ is
(a) H,
(b) halo,
(c) $NO_2$,
(d) $C_1$-$C_6$-alkyl,
(e) $C_1$-$C_6$-acyloxy,
(f) $C_3$-$C_7$-cycloalkyl,
(g) $C_1$-$C_6$-alkoxy,
(h) $-NHSO_2R^4$,
(i) hydroxy($C_1$-$C_4$-alkyl),
(j) aryl($C_1$-$C_4$-alkyl),
(k) $C_1$-$C_4$-alkylthio,
(l) $C_1$-$C_4$-alkylsulfinyl,
(m) $C_1$-$C_4$-alkylsulfonyl,
(n) $NH_2$,
(o) $C_1$-$C_4$-alkylamino,
(p) di($C_1$-$C_4$-alkyl)amino,
(q) polyfluoro-$C_1$-$C_4$-alkyl-,
(r) $-SO_2-NHR^9$,
(s) aryl as defined below,
(t) furyl,
(u) $C_2$-$C_6$-alkenyl, or
(v) $C_2$-$C_6$-alkynyl;

$R^4$ is H, aryl, heteroaryl or $C_1$-$C_6$ alkyl unsubstituted or substituted with aryl or heteroaryl;

$R^{4a}$ is aryl, or $C_1$-$C_6$-alkyl either unsubstituted or optionally substituted with aryl;

$R^5$ is

$$H,\ -\overset{R^4}{\overset{|}{C}}H-O-\overset{O}{\overset{\|}{C}}-R^{4a};$$

E is a single bond, $-NR^{13}(CH_2)_s-CH_3$, $-S(O)_x(CH_2)_s-CH_3$ where x is 0 to 2 and s is 0 to 5, -CH(OH)-, -O-, CO-;

$R^6$ is
(a) aryl either unsubstituted or substituted with 1 or 2 substituents selected from the group consisting of halo, -0-$C_1$-$C_4$-alkyl, $C_1$-$C_4$-alkyl, $-NO_2$, $-CF_3$, $-SO_2NR^9R^{10}$, -S-$C_1$-$C_4$-alkyl, -OH, $-NH_2$, $C_3$-$C_7$-cycloalkyl, and $C_3$-$C_{10}$-alkenyl;
(b) $C_1$-$C_6$-alkyl, $C_2$-$C_5$-alkenyl or $C_2$-$C_5$-alkynyl each of which can be substituted with a substituent selected from the group consisting of aryl, $C_3$-$C_7$-cycloalkyl, halo, $-CF_3$, $-CF_2CF_3$, $-NH_2$, -NH($C_1$-$C_4$-alkyl), $-OR^4$, -N($C_1$-$C_4$-alkyl)$_2$, $-NH-SO_2R^4$, $-COOR^4$, and $-SO_2NHR^9$;
(c) heteroaryl
(d) $C_3$-$C_7$-cycloalkyl;
(e) perfluoro-$C_1$-$C_4$-alkyl, or
(f) H;

$R^{7a}$ and $R^{7b}$ are independently

(a) H,
(b) $C_1$-$C_8$-alkyl unsubstituted or substituted with a substituent selected from the group consisting of -guanidino, $COOR^4$, $-CON(R^4)_2$, $-O-COR^4$, -aryl, -heteroaryl, -tetrazol-5-yl, $-CONHSO_2R^{22}$, $-SO_2NH$- heteroaryl, $-SO_2NHCOR^{22}$,$-PO(OR^4)_2$, $-PO(OR^4)R^9$, $-SO_2NH-CN$, and $-NR^{10}COOR^{22}$,
(c) -CO-aryl,
(d) $-C_3$-$C_7$-cycloalkyl,
(e) $-C_1$-$C_4$-perfluoroalkyl,
(f) $-COOR^4$,
(g) $-SO_3H$,
(h) $-NR^4R^{22}$,
(i) $-NR^4COR^{22}$,
(j) $-NR^4COOR^{22}$,
(k) $-NO_2$,
(l) $-N(R^4)SO_2R^{22}$,
(m) $-NR^4CONR^4R^{22}$,
(n)

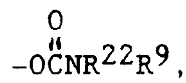

(o) -aryl or -heteroaryl,,
(p) $-NHSO_2CF_3$,
(q) $-SO_2NH$-heteroaryl,
(r) $-SO_2NHCOR^{22}$,
(s) $-CONHSO_2R^{22}$,
(t) $-PO(OR^4)2$,
(u) $-PO(OR^4)R^4$,
(v) $-COR^4$,
(w) $-NR^4SO_2NR^4R^{22}$,
(x) $-NR^4SO_2OR^{22}$
(y) $-CONR^4R^{22}$,
(z)

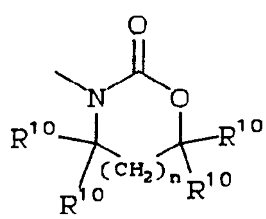

where n=0 or 1.
(aa)

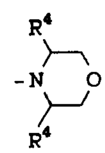

(bb)

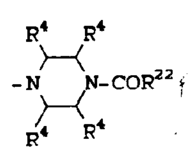

(cc)

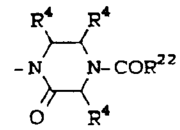

(dd)

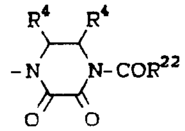

or
(ee)

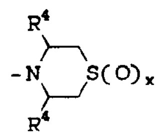

(ff)

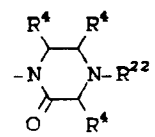

(gg)

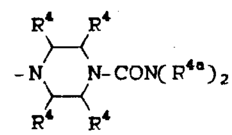

(hh)

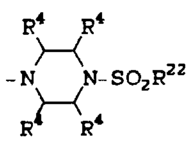

with the proviso that one of $R^{7a}$ and $R^{7b}$ is other than H;

$R^9$ is H, $C_1$-$C_5$-alkyl, aryl,heteroaryl or arylmethyl;

$R^{10}$ is H, $C_1$-$C_4$-alkyl;

$R^{11}$ is H, $C_1$-$C_6$-alkyl, $C_2$-$C_4$-alkenyl, $C_1$-$C_4$-alkoxy -$C_1$-$C_4$- alkyl, or

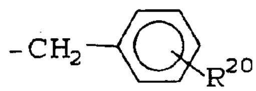

$R^{12}$ is -CN, $-NO_2$ or $-CO_2R^4$;
$R^{13}$ is H, ($C_1$-$C_4$-alkyl)CO-, $C_1$-$C_6$-alkyl, allyl, $C_3$-$C_6$-cycloalkyl, aryl or arylmethyl;
$R^{14}$ is H, $C_1$-$C_8$-alkyl, $C_1$-$C_8$-perfluoroalkyl, $C_3$-$C_6$-cycloalkyl, aryl or arylmethyl;
$R^{15}$ is H, $C_1$-$C_6$-alkyl;
$R^{16}$ is H, $C_1$-$C_6$-alkyl, $C_3$-$C_6$-cycloalkyl, aryl or arylmethyl;
$R^{17}$ is $-NR^9R^{10}$, $-OR^{10}$, $-NHCONH_2$, $-NHCSNH_2$,

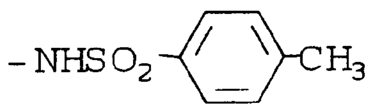 or 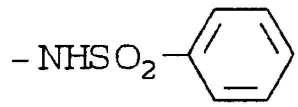 ;

$R^{18}$ and $R^{19}$ are independently $C_1$-$C_4$-alkyl or taken together are $-(CH_2)_q-$ where q is 2 or 3;
$R^{20}$ is H, $-NO_2$, $-NH_2$, -OH or $-OCH_3$;
$R^{22}$ is
(a) aryl,
(b) heteroaryl,
(c) $C_3$-$C_7$-cycloalkyl,
(d) $C_1$-$C_6$-alkyl unsubstituted or substituted with a substituent selected from the group consisting of aryl, heteroaryl, -OH, -SH, $C_1$-$C_4$-alkyl, -O($C_1$-$C_4$-alkyl),-S($C_1$-$C_4$-alkyl), $-CF_3$, halo, $-NO_2$, $-CO_2H$, $CO_2$-($C_1$-$C_4$-alkyl), $-NH_2$, -NH($C_1$-$C_4$-alkyl), -N($C_1$-$C_4$-alkyl)$_2$, $-PO_3H_2$, -PO(OH)(O-$C_1$-$C_4$-alkyl), $-PO(OR^4)R^9$;
(e) perfluoro-$C_1$-$C_4$-alkyl;
X is
(a) a carbon-carbon single bond,
(b) -CO-,
(c) -O-,
(d) -S-,
(e)

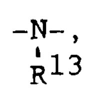

(f)

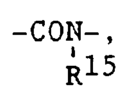

(g)

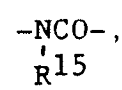

(h) $-OCH_2-$,
(i) $-CH_2O-$,
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,

(p) -CH=CH-,
(q) -CF=CF-,
(r) -CH=CF-,
(s) -CF=CH-,
(t) $-CH_2CH_2-$,
(u) $-CF_2CF_2-$,
(v)

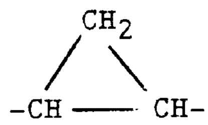 or 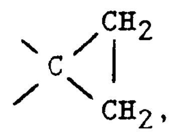

(w)

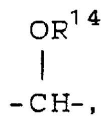

(x)

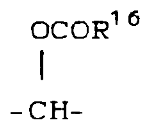

(y)

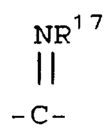 ,

or
(z)

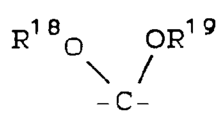 ;

r is 1 or 2; and

The term "aryl" means phenyl or naphthyl either unsubstituted or substituted with one, two or three substitutes selected from the group consisting of halo, $C_{1-4}$-alkyl, $C_{1-4}$-alkoxy, $NO_2$, $CF_3$, $C_{1-4}$-alkylthio, OH, $-N(R^6)_2$, $-CO_2R^6$, $C_{1-4}$-polyfluoroalkyl, $C_{3-6}$-polyfluorocycloalkyl, and tetrazol-5-yl.

The term "heteroaryl" means a five or six membered aromatic heterocycle comprising from 1 to 3 heteroatoms of O, N and S such as imidazole thiazole, thiophem pyrazine, pyridine pyridazine, pyrimidine or the like either unsubstituted or substituted with up to three substitutents selected from the group consisting of -OH, -SH, $-C_{1-4}$-alkyl, $-C_{1-4}$-alkoxy, $-CF_3$, halo, $-NO_2$, $-CO_2R^6$,$-N(R^6)_2$ and a fused benzo group.

The term "halo" means -Cl, Br, -I or -F.

The term "alkyl", "alkenyl", "alkynyl" and the like include both the straight chain and branched chain species of these generic terms wherein the number of carbon atoms in the species permit. Unless otherwise noted, the specific names for these generic terms shall mean the straight chain species. For example, the term "butyl" shall mean the normal butyl substituent, n-butyl.

One embodiment of the compounds of formula (I) are those compounds wherein:

$R^1$ is

(a) -COOH,

(b)

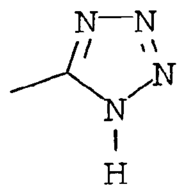

,

(c) $-NH-SO_2CF_3$;

(d) $-SO_2NH$-heteroaryl,

(e) $-CH_2SO_2NH$-heteroaryl,

(f) $-SO_2NH-CO-R^{22}$,

(g) $-CH_2SO_2NH-CO-R^{22}$,

(h) $-CONH-SO_2R^{22}$,

(i) $-CH_2CONH-SO_2R^{22}$,

(j) $-NHSO_2NHCO-R^{22}$,

(k) $-NHCONHSO_2-R^{22}$,

$R^{2a}$ is H;

$R^{2b}$ is H, F, Cl, $CF_3$, $C_1$-$C_6$-alkyl, $C_2$-$C_6$-alkenyl, $C_2$-$C_6$-alkynyl, or aryl;

$R^{3a}$ is H;

$R^{3b}$ is H, F, Cl, $CF_3$, $C_1$-$C_4$-alkyl, $C_2$-$C_4$-alkenyl, $C_2$-$C_4$-alkynyl, $C_5$-$C_6$-cycloalkyl, $-COOCH_3$, $-COOC_2H_5$, $-SO_2-CH_3$, $NH_2$, $-N(C_1\text{-}C_4\text{-alkyl})_2$ or $-NH-SO_2CH_3$;

E is a single bond, -O- or -S-;

$R^6$ is

(a) $C_1$-$C_5$ alkyl unsubstituted or substituted with a substituent selected from the group consisting of $C_3$-$C_5$-cycloalkyl, Cl, $CF_3$, $CCl_3$, $-O-CH_3$, $-OC_2H_5$, $-S-CH_3$, $-S-C_2H_5$, phenyl, or F;

(b) $C_2$-$C_5$-alkenyl or $C_2$-$C_5$-alkynyl; or,

(c) $C_3$-$C_5$-cycloalkyl;

$R^{7a}$ and $R^{7b}$ are independently

(a) H,

(b) $C_1$-$C_8$-alkyl unsubstituted or substituted with COOR, $OCOR^{4a}$, OH, aryl, or $-(CH_2)_{1-4}R^4$;

(c) $-NO_2$,

$R^4O$

(d) $-N-C-R^{22}$,

(e) $-CONR^4R^{22}$,

(f)

$$\overset{O}{\phantom{x}}\qquad -NR^4-\overset{\displaystyle O}{C}-O-R^{22},$$

(g) $-NR^4R^{22}$,

(h) halo,

(i) $-CF_3$,

(j) $-CO_2R^4$,

(k) -CO-aryl as defined above,

(l) $-N(R^4)SO_2R^{22}$,

(m) aryl or heteroaryl as defined above,

(n) $-NR^4CONR^4R^{22}$,
(o) $-N(R^4)SO_2N(R^4)R^{22}$;
(p)

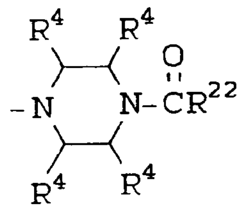

(q)

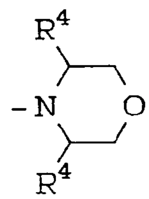

X is a single bond;
r is one
with the proviso that one of $R^{7a}$ and $R^{7b}$ is other than H.

In a class of this embodiment are those compounds of Formula (I) wherein:
$R^1$ is
(a) -COOH,
(b)

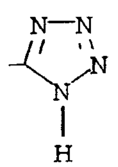

(c) $-NH-SO_2-CF_3$,
(d) $-SO_2NH$-heteroaryl as defined above.
(e) $-SO_2NH-CO-R^{22}$,
(f) $-CONH-SO_2R^{22}$.
E is a single bond;
r is one,
$R^{2a}$, $R^{2b}$, $R^{3a}$ and $R^{3b}$ are each H, $-C_1-C_6$-alkyl, $-C_2-C_6$-alkenyl, $-C_2-C_6$-alkynyl, -Cl, -F, $-NO_2$, $-CF_3$;
$R^6$ is $-C_1-C_4$-alkyl, -cyclopropyl, $-CH_2CH_2CH_2CF_3$, $-CH_2CH_2CF_3$, $-C_2-C_5$-alkenyl, -cyclopropylmethyl.
$R^{7a}$ and $R^{7b}$ are each independently H, $-C_1-C_4$-alkyl, $-NO_2$, $-NR^4R^{22}$, $-NR^4COOR^{22}$, $-CH_2COOR^{4a}$, $-S(O)_x-R^{22}$ alkyl, $NR^4CONR^4R^{22}$, $CH_2OCO(C_1-C_4$-alkyl), $NR^4COR^{22}$, $CO_2R^4$, -F, $-CH_2Ph$, $-CONR^4R^{22}$.

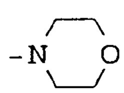 , 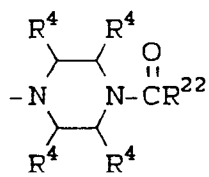

with the proviso that one of $R^{7a}$ and $R^{7b}$ is other than H.

In a subclass are those compounds of Formula (I) wherein:

$R^1$ is
(a) COOH,
(b)

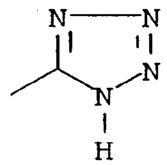

(c) $-SO_2NHCOR^{22}$,
(d) $-CONHSO_2R^{22}$,
(e) $-NHSO_2CF_3$;
$R^{2a}$, $R^{2b}$, $R^{3a}$ and $R^{3b}$ are each H, $-C_1$-$C_4$-alkyl, -Cl or F;
$R^{7a}$ or $R^{7b}$ are: H, halo, $NR^4R^{22}$, $C_1$-$C_4$ alkyl, $NR^4COR^{22}$, $NR^4COOR^{22}$, $NR^4CONR^4R^{22}$

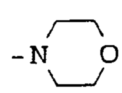
,
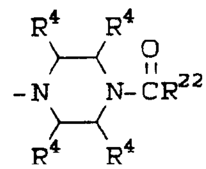

Exemplifying this subclass are the following compounds:
(1) 2-n-Butyl-3-[(2′-carboxybiphen-4-yl)methyl]pyrazino[2,3-d]pyrimidin-4(3H)-one;
(2) 2-n-Butyl-3-[(2′-(tetrazol-5-yl)biphen-4-yl)methyl]pyrazino[2,3-d]pyrimidin-4(3H)-one;
(3) 2-n-Butyl-3-[2′-(carboxybiphen-4-yl)methyl]pyrazino[2,3-d]pyrimidin-4(3H)-one;
(4) 2-n-Butyl-3-[(2′-(tetrazol-5-yl)biphen-4-yl)methyl]pyrazino[2,3-d]pyrimidin-4(3H)-one;
(5) 2-n-Butyl-7-isopropyl-3-[(2′-(tetrazol-5-yl)biphen-4-yl)methyl]pyrazino[2,3-d]pyrimidin -4(3H)-one;
(6) 6-Amino-2-n-Butyl-3-[(2′-(tetrazol-5-yl)biphen-4-yl)methyl]pyrazino[2,3-d]pyrimidin -4(3H)-one;
(7) 6-Acetamido-2-n-Butyl-3-[(2′-(tetrazol-5-yl)biphen-4-yl)methyl]pyrazino[2,3-d]pyrimidin -4(3H)-one;
(8)- 2-n-Butyl-6-methyl-3-[(2′-(tetrazol-5-yl)biphen-4-yl)methyl]pyrazino[2,3-d]pyrimidin-4(3H)-one;
(9) 2-n-Butyl-3-[(2′-(tetrazol-5-yl)biphen-4-yl) methyl]-6-thiomethylpyrazino[2,3-d]pyrimidin -4(3H)-one;
(10) 2-n-Butyl-7-carboxy-3-[(2′-(tetrazol-5-yl)biphen-4-yl)methyl]pyrazino[2,3-d]pyrimidin -4(3H)-one;
(11) 2-n-Butyl-7-(N-isopropylcarbamoyl)amino-3[(2′-(tetrazol-5-yl)biphen-4-yl)methyl]pyrazino-[2,3-d]pyrimidin-4(3H)-one;
(12) 2-n-Butyl-6-(N-isobutyloxycarbonyl)amino-3[(2′-(tetrazol-5-yl)biphen-4-yl)methyl]pyrazino-[2,3-d]pyrimidin-4(3H)-one;
(13) 2-n-Butyl-6-[N-(morpholin-4-yl)carbamoyl)-N-methyl]amino-3-[(2′-tetrazol-5-yl)biphen-4-yl)-methyl]pyrazino[2,3-d]pyrimidin-4(3H)-one;
(14) 2-n-Butyl-6-(N-isopropyloxycarbonyl-N-methyl) amino-3-[(2′-tetrazol-5-yl)biphen-4-yl)methyl]pyrazino[2,3-d]pyrimidin-4(3H)-one;
(15) 6-(N-Benzyloxycarbonyl-N-methyl)amino-2-n-butyl -3-[(2′-(tetrazol-5-yl)biphen-4-yl)methyl]- pyrazino[2,3-d]pyrimidin-4(3H)-one;
(16) 3-[(2′-(N-Benzoylsulfonamido)biphen-4-yl)]methyl-2-n-butyl-6-(N-isopropyloxycarbonyl-N-benzyl)amino-pyrazino[2,3-d]pyrimidin-4(3H)-one;
(17) 2-n-Butyl-6-(N-isopropyloxycarbonyl-N-methyl)amino-3-[(2′-(N-trifluoromethylsulfony, 1-carboxamido)biphen-4-yl)methyl]pyrazino- [2,3-d]- pyrimidin-4(3H)-one;
(18) 2-n-Butyl-3-[(2′-(tetrazol-5-yl)biphen-4-yl)methyl]pyrazino[2,3-d]pyrimidin-4(3H)-one;
(19) 6-[N-Benzyl-N-n-butyloxycarbonyl]amino-2 -propyl-3-[(2′-(tetrazol-5-yl))biphen-4-yl) methyl]-pyrazino[2,3-d]pyrimidin-4(3H)-one;
(20) 2-n-Butyl-6-(N-methyl-N-isobutyloxycarbonyl) amino-3-[(2′-(tetrazol-5-yl)biphen-4-yl)methyl]pyrazino[2,3-d]pyrimidin-4(3H)-one;
(21) 6-(N-Benzyl-N-butanoyl)amino-2-n-propyl-3[(2′-(tetrazol-5-yl)biphen-4-yl)methyl]pyrazino[2,3-d]pyrimidin-4(3H)-one;
(22) 6-(N-Benzoyl-N-n-pentyl)amino-2-n-propyl-3[(2′-(tetrazol-5-yl)biphen-4-yl)methyl]pyrazino[2,3-d]pyrimidin-4(3H)-one;

(23) 6-(N-(p-Chloro)benzoyl-N-n-pentyl)amino-2-n-propyl-3-[(2′-(tetrazol-5-yl)biphen-4-yl)methyl]pyrazino[2,3-d]pyrimidin-4(3H)-one;
(24) 6-(N-(p-Chloro)benzoyl-N-isobutyl)amino-2-n-propyl-3-[(2′-(tetrazol-5-yl)biphen-4-yl)methyl] pyrazino[2,3-d]pyrimidin-4(3H)-one;
(25) 6-(N-n-Propyl-N-isobutyloxycarbonyl)amino-2-n-propyl-3-[(2′-(tetrazol-5-yl)biphen-4-yl)methyl]-pyrazino[2,3-d]pyrimidin-4(3H)-one;
(26) 6-(N-Benzoyl-N-n-pentyl)amino-3-[(2′-(N-benzoyl-sulfonamido)biphen-4-yl)methyl]-2-n-propyl pyrazino[2,3-d]pyrimidin-4(3H)-one;
(27) 2-n-Butyl-6-(N-methyl-N-isobutyloxycarbonyl) amino-3-[(2′-(tetrazol-5-yl)biphen-4-yl)methyl]pyrazino[2,3-d]pyrimidin-4(3H)-one;
(28) 6-(N-Benzyl-N-butanoyl)amino-2-n-propyl-3[(2′-(tetrazol-5-yl)biphen-4-yl)methyl]pyrazino[2,3-d]pyrimidin-4(3H)-one;
(29) 6-(N-(p-Chloro)benzoyl-N-n-pentyl)amino-2-n-propyl-3-[(2′-(tetrazol-5-yl)biphen-4-yl)methyl]pyrazino[2,3-d]pyrimidin-4(3H)-one;
(30) 6-(N-n-Propyl-N-isobutyloxycarbonyl)amino-2-n-propyl-3-[(2′-(tetrazol-5-yl)biphen-4-yl)methyl]pyrazino[2,3-d]pyrimidin-4(3H)-one;
(31) 6-(N-Benzoyl-N-n-pentyl)amino-3-[(2′-(N-benzoyl -sulfonamido)biphen-4-yl)methyl]-2-n-propylpyrazino[2,3-d]pyrimidin-4(3H)-one;
(32) 2-N-butyl-3-[(2′-(N-cyclopropylcarbonyl-sulfonamido)biphen-4-yl)methyl]-6-(N-cyclopropylcarbonyl-piperazin-1-yl)-pyrazino- [2,3-d]-pyrimidin-4(3H)-one.
(33) 6-(N-acetyl-piperazin-1-yl)-2-n-propyl-3[(2′-(tetrazol-5-yl)biphen-4-yl)methyl]-pyrazino[2,3-d]pyrimidin-4(3H)-one.
(34) 6-(N-benzyl-N-benzoyl)-2-methyl-3-[(2′-(tetrazol-5-yl)biphen-4-yl)methyl]-pyrazino[2,3d]-pyrimidin-4(3H)-one;
(35) 6-(n-benzyl-N-benzoyl)-2-isopropyl-3-[(2′(tetrazol-5-yl)biphen-4-yl)methyl]-pyrazino- [2,3-d]pyrimidin-4(3H)-one, and
(36) 2-ethyl-6-(N-(2-thienylcarbonyl)-N-benzyl)3-[(2′-(tetrazol-5-yl)biphen-4-yl)methyl]pyrazino[2,3-d]pyrimidin-4(3H)-one.

The compounds of Formula (I) can be synthesized using the reactions and techniques described below. The reaction are performed in a solvent appropriate to the reagents and materials employed and suitable for the transformation being effective. It is understood by those skilled in the art of organic synthesis that the functionality present on the heterocycle and other parts of the structure should be consistent with the chemical transformations proposed. Depending upon the reactions and techniques employed, this may involve changing the order of the synthetic steps, the use of required protecting groups followed by deprotection, and, depending upon the particular pyrimidinone fused heterocycle being formed, the use of different strategies may be employed regarding the cyclization steps and the particular starting materials utilized.

ABBREVIATIONS USED IN REACTION SCHEMES

Reagents:

| | |
|---|---|
| NBS | N-bromosuccinimide |
| AIBN | azo(bis)isobutyronitrile |
| DDQ | dichlorodicyanoquinone |
| $Ac_2O$ | acetic anhydride |
| TEA | triethylamine |
| DMAP | 4-dimethylaminopyridine |
| $PPh_3$ | triphenylphosphine |
| TFA | trifluoroacetic acid |
| TMS-Cl | trimethylsilyl chloride |
| Im | imidazole |
| AcSK | potassium thioacetate |
| p-TsOH | p-toluenesulfonic acid |

Solvents:

| | |
|---|---|
| $Et_2O$ | diethyl ether |
| DMF | dimethylformamide |

| | |
|---|---|
| HOAc (AcOH) | acetic acid |
| EtOAc (EtAc) | ethyl acetate |
| Hex | hexane |
| THF | tetrahydrofuran |
| DMSO | dimethylsulfoxide |
| MeOH | methanol |
| iPrOH | isopropanol |
| DBU | 1,8-diazabicyclo-[5.4.0] undec-7-ene |
| $Me_3SnCl$ | trimethylstannyl chloride |

Others:

| | |
|---|---|
| rt | room temperature |
| TBDMS | t-butyldimethylsilyl |
| OTf | $OSO_2CF_3$ |
| OTs | $OSO_2$-(4-methyl)phenyl |
| OMs | $OSO_2CH_3$ |
| Ph | phenyl |
| FAB-MS (FABMS) | Fast atom bombardment mass spectroscopy |
| NOE | Nuclear Overhauser Effect |
| $SiO_2$ | silica gel |
| trityl | triphenylmethyl. |
| s.b. | single bond |

Compounds of Formula I may be prepared via the intermediate 1 by the alkylation of 2-substituted-pyrazino[2,3-d]pyrimidinones 2 with biphenyl methyl acid precursors 3 in the presence of a base such as sodium hydride (Scheme 1).

Deprotection of the $R^1$ protecting group of 1 or the modification of the precursor to the $R^1$ group will give rise to compounds of Formula I. For example if $R^1$ is nitrile and a tetrazole is desired, trimethyltin azide will convert the intermediate 1 into I where $R^1$ is tetrazole.

## Scheme 1

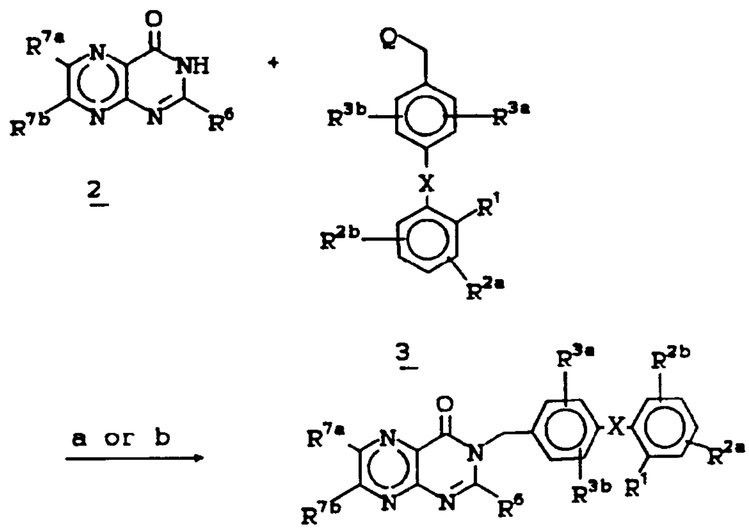


1 where r = 1 $R^1$ is protected

I where r = 1 $R^1$ is unprotected

Q= halogen, -O-tosyl, O-mesyl

a. NaH, DMF

b. (i) 2 $((CH_3)_3Si)_2NH$ or $(CH_3)_3SiCl/Et_3N$

(ii) 3, $F^-$

Alternatively a route to compounds 1 where r >1 is available by condensation of compounds of structure 4 with an amino biphenyl methyl amine such as 5.[1] 4 may be prepared by heating the amino pyrazine carboxylic acid with excess acid chloride or anhydride. The precursor 2 may be prepared by heating 4 with ammorium carbonate to give the 2-substituted-pyraziro[2,3-d]pyrimidinores 2.

[1]Irwin, W. J.; Wibberley, D., J. A New Pteridine Synthesis. Tetrahedron Lett. 1972, 32, 3359-3360.

Scheme 2

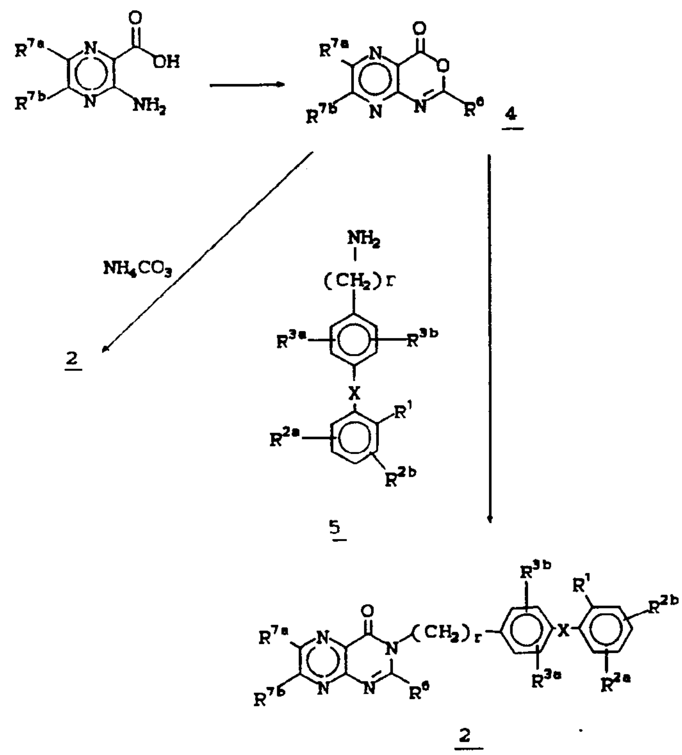

4

2

5

2

1 where r >1 and $R^1$ is protected
I where r >1 and $R^1$ is unprotected

A further route to compounds of structure 2 is available by the base catalysed cyclizaton of the 2-amino-pyrazine-3-carboxamide 6.[2] Compounds 6 may be prepared by several alternative routes including, but not limited to, the hydrolysis of nitriles of structure 7. This route, under basic conditions, results in the direct conversion of 7 into 2. The acylation of the amino amide 8 may also give rise to 6. Amides of structure 8 are available from amino carboxylic acid pyrazines 9 by esterification followed by aminolysis. Compounds of structure 9 are commercially available or must be prepared by well established means.[3] Compounds of structure 7 may be prepared by acylation of the amino nitriles such as 10 which may in turn be prepared by well established routes.

[2]Albert, A. Pteridine Studies. part 46. 2-alkyl-pteridin-4-ones from 2-aminopyrazine-3-carboxamide and its Drivatives. J. Chem. Soc. 1979, 1575.
[3]Porter, A., E., A. Pyrazines and their Benzo derivatives. Comprehensive Heterocyclic Chemistry, Boulton, A., J. and McKillop, A., Eds.;Pergamon Press: New York, 1984; 157-197.

Scheme 3

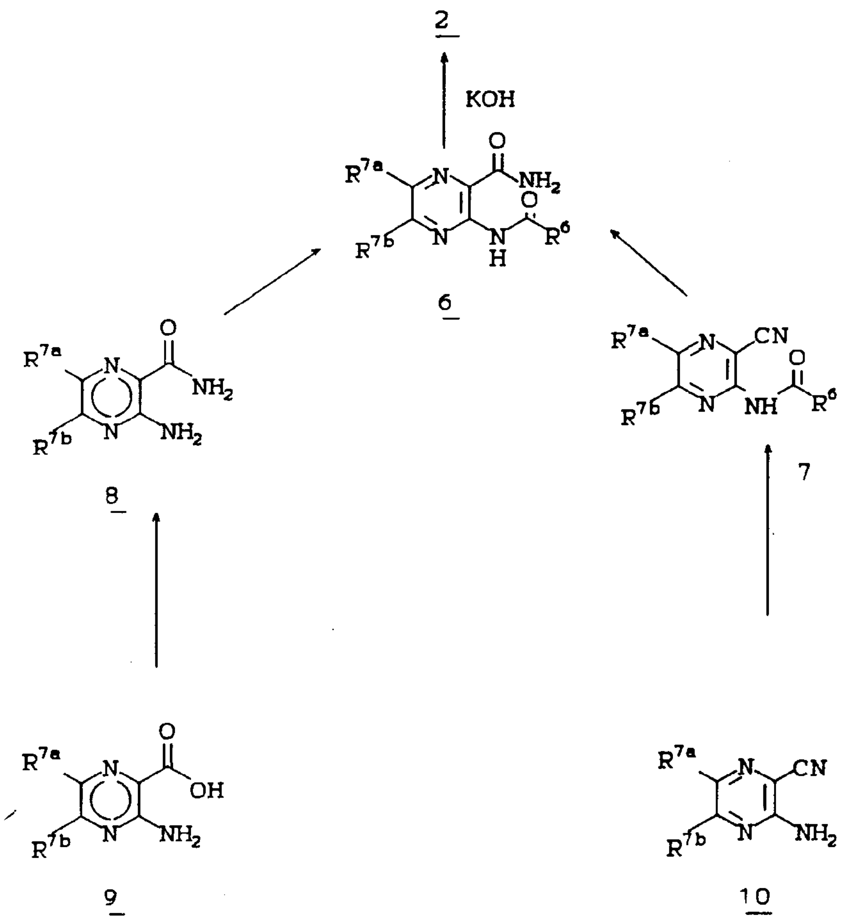


The benzyl halides (3) including the more preferred alkylating agents (11a and 11b, Scheme 4) can be prepared as described in European Patent Applications 253,310 and 291,969 and the references cited therein. However, a preferred method to prepare the biphenyl precursors 12a, 12b and 12C, using Ni(0) or Pd(O) catalyzed cross-coupling reaction [E. Negishi, T. Takahashi, and A. O. King, Org. Synthesis, 66, 67 (1987)], is outlined in Scheme 4. As shown in Scheme 4, treatment of 4-bromotoluene (13) with t-BuLi, followed by the addition of a solution of $ZnCl_2$, produces the organo-zinc compound (14). Compound (14) is then coupled with 15a or 15b in the presence of $Ni(PPh_3)_2Cl_2$ catalyst to produce the desired biphenyl compound 12a or 12b ($PPh_3$=triphenylphosphine). Similarily, 1-iodo-2-nitro-benzene (15c) is coupled with organo-zinc compound 14 in the presence of $Pd(PPh_3)_4$ catalyst [prepared by treating $Cl_2Pd(PPh_3)_2$ with $(i\text{-}Bu)_2A1H$ (2 equiv.)] to give the biphenyl compound 12c. These precursors, 12a, 12b and 12c, are then transformed into halomethylbiphenyl derivatives 11a, 11b and 11c, respectively, according to procedures described in European Patent Applications 253,310 and 291,969.

SCHEME 4

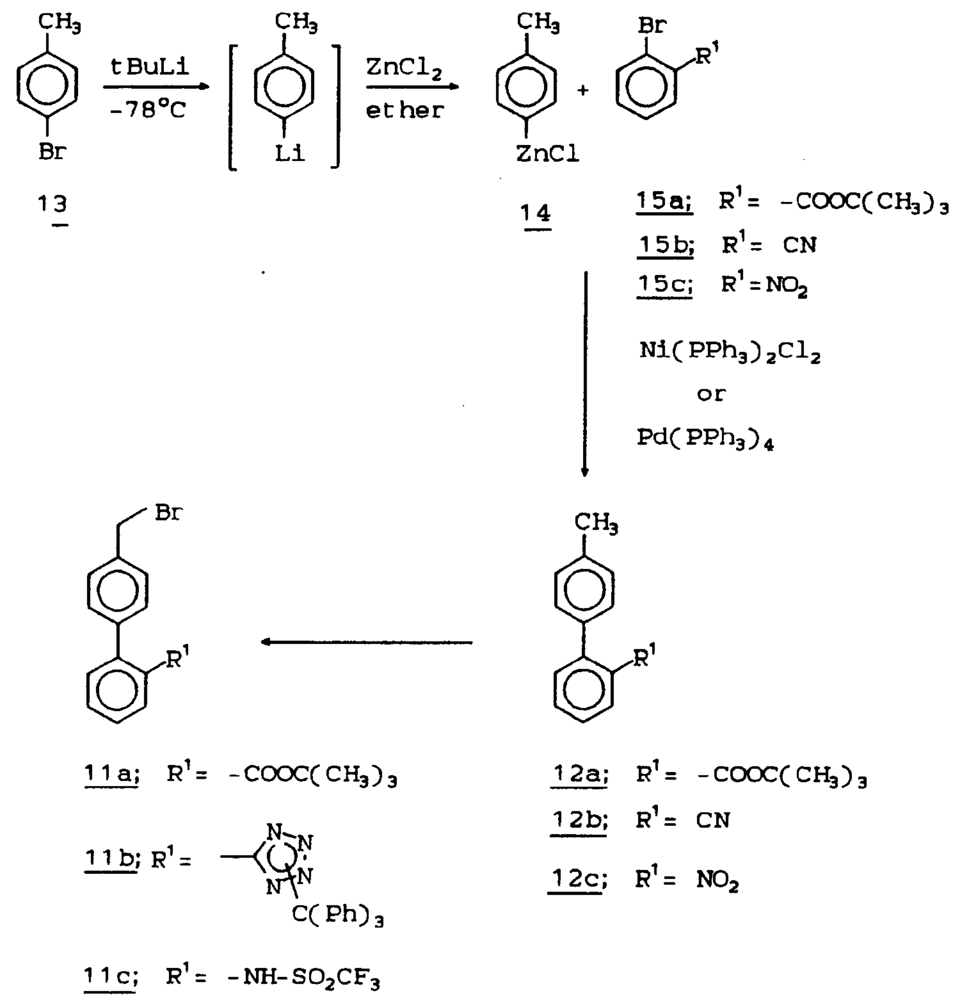


13

14

15a; $R^1$ = $-COOC(CH_3)_3$

15b; $R^1$ = CN

15c; $R^1$ = $NO_2$

11a; $R^1$ = $-COOC(CH_3)_3$

11b; $R^1$ =

11c; $R^1$ = $-NH-SO_2CF_3$

12a; $R^1$ = $-COOC(CH_3)_3$

12b; $R^1$ = CN

12c; $R^1$ = $NO_2$

When there is additional substitution on the second phenyl ring ($R^{2a}$, $R^{2b}$ = hydrogen) the preferred method to prepare the biphenyl precursors 12d and 12e, using the Pd(0) catalyzed cross-coupling reaction [J. K. Stille, Angew, Chem. Int. Ed. Engl., 25, 508 (1986)], is outlined in reaction Scheme 5. As shown in Scheme 5, p-tolyltrimethyltin (16) is coupled with 15d or 15e in refluxing toluene in the presence of 5 mole % of $Pd(PPh_3)_4$ to produce the desired biphenyl compounds 12d and 12e. Table I illustrates the synthetic utility of this protocol. Compounds 12d ($R^2 = NO_2$) and 12e ($R^2 = NO_2$) could be converted to their respective chlorides by catalytic hydrogenation, diazotization and treatment with copper (I) chloride. The biphenyl fluorides which could not be obtained by direct coupling to a fluoro arylbromide were prepared from 12d ($R^2 = NO_2$) and 12e, ($R^2 = NO_2$) via reduction, formation of the diazonium tetrafluoroborate salt and thermal decomposition. These precursors 12d ($R^2$ = $NO_2$ or F or Cl) and 12e ($R^2$ = $NO_2$ or F or Cl) are then transformed into the halomethyl biphenyl derivatives 11d and 11e, respectively according to the procedures described in European Patent Applications 253,310 and 292,969.

Biphenyl Synthesis Table I

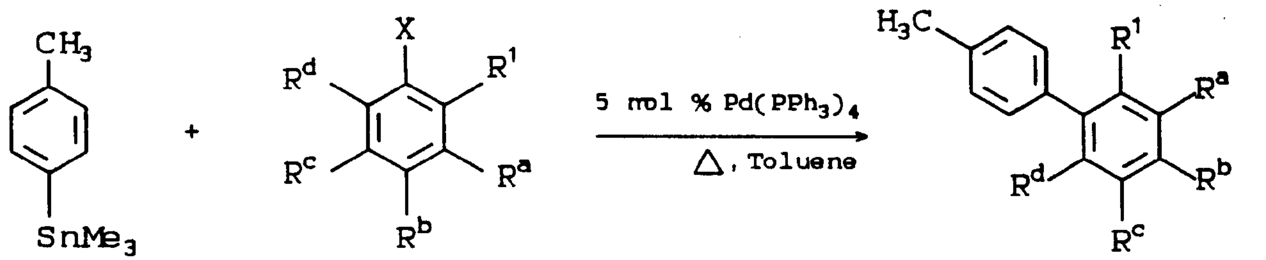


16 (15d, 15e) (12d, 12e)

| X | $R^1$ | $R^a$ | $R^b$ | $R^c$ | $R^d$ | Product ($R^a$) | Rf (solvent) | Yield |
|---|---|---|---|---|---|---|---|---|
| Br | $CO_2Me$ | $NO_2$ | H | H | H | 12d (3'-nitro) | 0.35(15:1 Hex/EtOAc) | 71% |
| Br | CN | H | $NO_2$ | H | H | 12e (4'-nitro) | 0.62(2x 6:1 Hex/EtOAc) | 74% |
| Br | $CO_2Me$ | H | F | H | H | 12d (4'-fluoro) | 0.43(15:1 Hex/EtOAc) | 83% |
| Cl | $CO_2Me$ | H | H | $NO_2$ | H | 12d (5'-nitro) | 0.22(15:1 Hex/EtOAc) | 70% |
| Br | $CO_2Me$ | H | H | H | $NO_2$ | 12d (6'-nitro) | 0.24(15:1 Hex/EtOAc) | 79% |
| Br | CN | H | F | H | H | 12e (4'-fluoro) | 0.44(15:1 Hex/EtOAc) | 64% |
| Cl | CN | H | H | F | H | 12e (5'-fluoro) | 0.40(15:1 Hex/EtOAc) | 62% |

33

## REACTION SCHEME 5

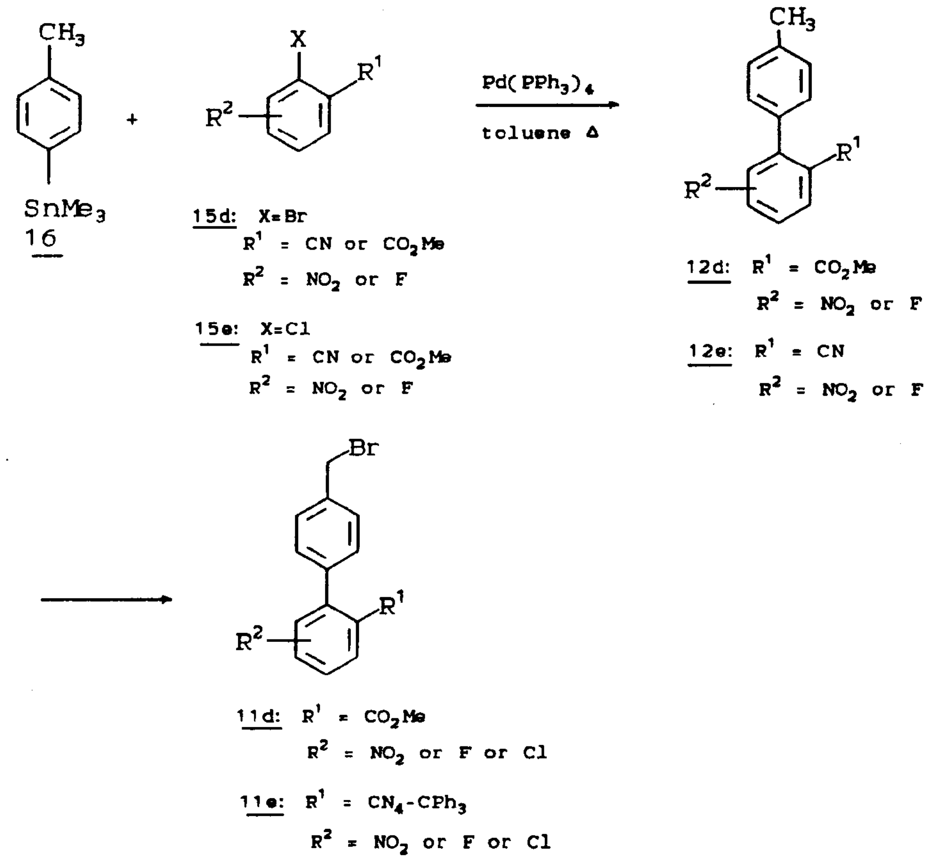


16

15d: X=Br
$R^1$ = CN or $CO_2Me$
$R^2$ = $NO_2$ or F

15e: X=Cl
$R^1$ = CN or $CO_2Me$
$R^2$ = $NO_2$ or F

12d: $R^1$ = $CO_2Me$
$R^2$ = $NO_2$ or F

12e: $R^1$ = CN
$R^2$ = $NO_2$ or F

11d: $R^1$ = $CO_2Me$
$R^2$ = $NO_2$ or F or Cl

11e: $R^1$ = $CN_4$-$CPh_3$
$R^2$ = $NO_2$ or F or Cl

Compounds of formula I where $R^1$ is $-CONHSO_2R^{22}$ (where $R^{22}$ = alkyl, aryl or heteroaryl) may be prepared from the corresponding carboxylic acid derivatives (17) as outlined in Scheme 6. The carboxylic acid (17), obtained as described in Scheme 1, can be converted into the corresponding acid chloride by treatment with refluxing thionyl chloride or preferably with oxalyl chloride and a catalytic amount of dimethylformamide at low temperature [A.W. Burgstahler, L.O. Weigel, and C.G. Shaefer-Synthesis, 767, (1976)]. The acid chloride then can be treated with the alkali metal salt of $R^{22}SO_2NH_2$ to form the desired acylsulfonamide (18). Alternatively, these acylsulfonamides may be prepared from the carboxylic acids using N,N-diphenylcarbamoyl anhydride intermediates [F.J. Brown et al, European Patent Application, EP 199543; K.L. Shepard and W. Halczenko- J. Het. Chem., 16, 321 (1979)]. Preferably the carboxylic acids can be converted into acyl-imidazole intermediates, which then can be treated with an appropriate aryl or alkylsulfonamide and diazabicycloundecane (DBU) to give the desired acylsulfonamide 18 [J.T. Drummond and G. Johnson, Tetrahedron. Lett., 28, 1653 (1988)].

SCHEME 6

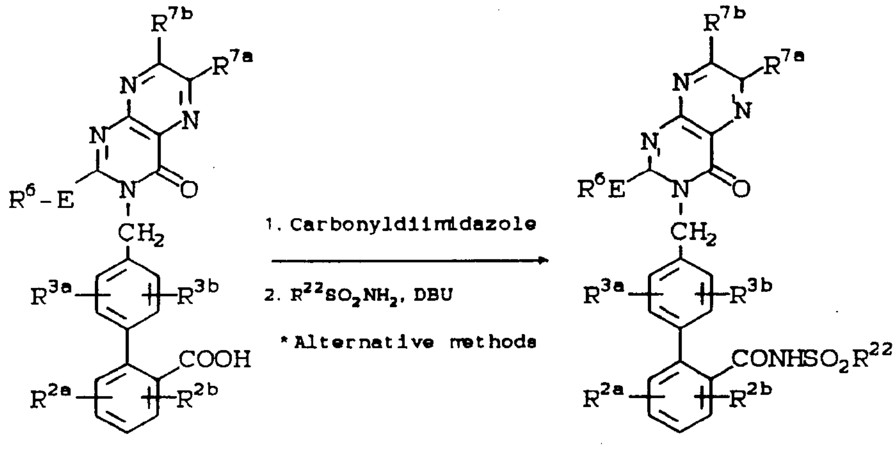


17    18

*Alternative Methods:

a) (i) $SOCl_2$, reflux

(ii) $R^{22}SO_2NH^-M^+$ (where M is Na or Li)

b) (i) $(COCl)_2$-DMF, -20°C

(ii) $R^{22}SO_2NH^-M^+$

c) (i) N(N,N-Diphenylcarbamoyl)pyridinium chloride/ aq. NaOH

(ii) $R^{22}SO_2NH^-M^+$.

Compounds of formula I where $R^1$ is $SO_2NHCOR^{22}$ may be prepared as outlined in Scheme 7. The nitro compound, for example 12c (prepared as described in Scheme 4), can be reduced to the corresponding amino compound and converted into aromatic diazonium chloride salt, which then can be reacted with sulfur-dioxide in the presence of a copper (II) salt to form the corresponding arylsulfonyl chloride 19 [H. Meerwein, G. Dittmar, R. Gollner, K. Hafner, F. Mensch and O. Steifort, Chem. Ber., 90, 841 (1957); A.J. Prinsen and H. Cerfontain, Recueil, 84, 24 (1965); E.E. Gilbert, Synthesis, 3 (1969) and references cited therein]. The sulfonyl chloride can be reacted with ammonia in aqueous solution or in an inert organic solvent [F.H. Bergheim and W. Baker, J. Amer. Chem. Soc, 66, (1944), 1459], or with dry powdered ammonium carbonate, [E.H. Huntress and J.S. Autenrieth, J. Amer. Chem. Soc., 63 (1941), 3446; E.H. Huntress and F.H. Carten, J. Amer. Chem. Soc., 62, (1940), 511] to form the sulfonamide 20. The sulfonamide must then be protected preferably with the triphenylmethyl group by reaction with triphenylmethylchloride and triethylamine to give 21. The benzyl bromide 22 may be prepared from the sulfonamide 21 as outlined in Scheme 5, and then can be reacted with an alkali metal salt of an appropriate heterocyclic compound to form the key sulfonamide 23. The sulfonamide 23 may then be acylated under appropriate conditions to give 24. The sulfonamide 23 may be also prepared from the

aromatic sulfonyl chloride 26 by treatment with ammonia. Compound 26 may be prepared from the aryl amine 25 as outlined in Scheme 8.

SCHEME 7

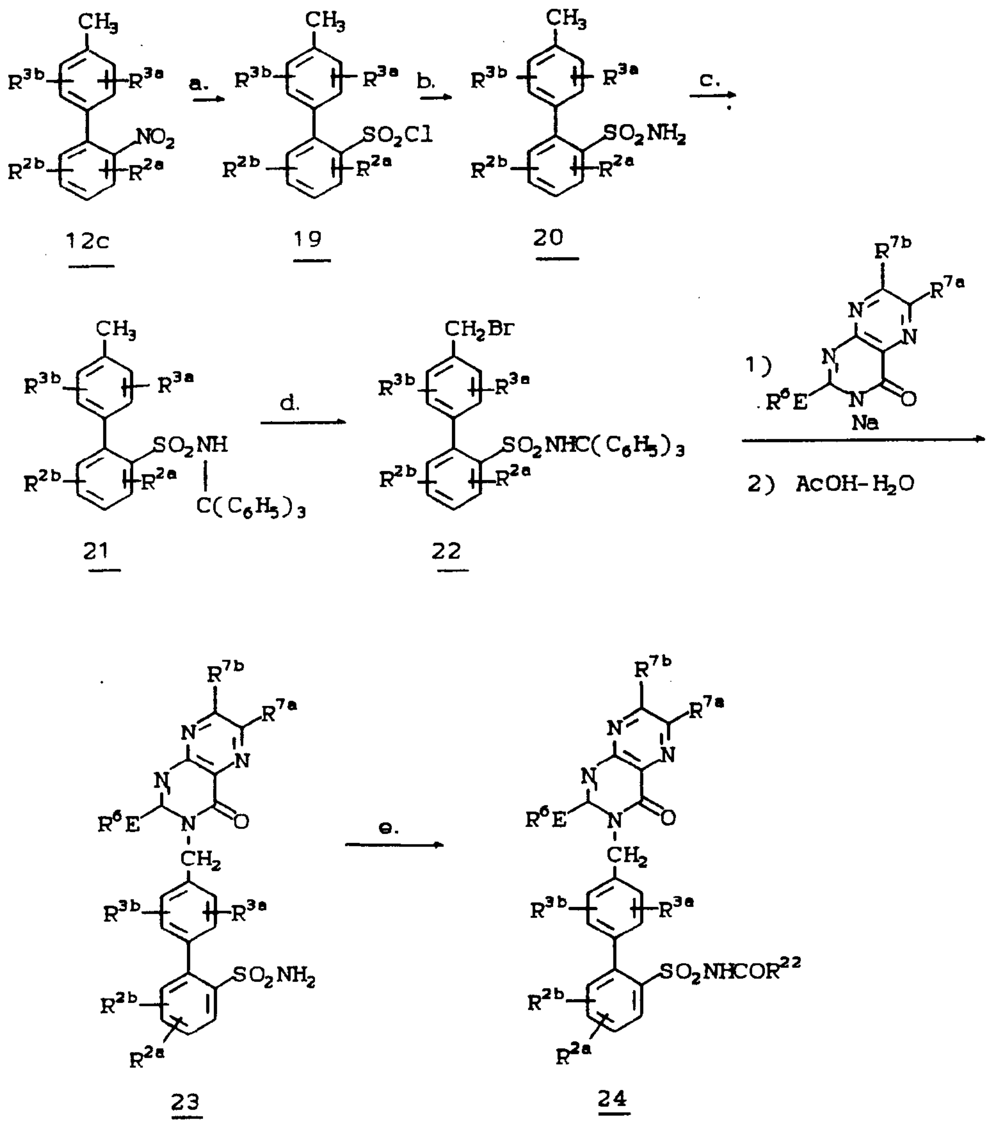


12c 19 20 21 22 23 24

SCHEME 7 (CONT'D)

a. (i) $H_2/Pd-C$,
(ii) $NaNO_2-HCl$,
(iii) $SO_2$, AcOH, $CuCl_2$
b. $NH_3$ or $(NH_4)_2CO_3$
c. $(C_6H_5)_3CCl$, $Et_3N$, $CH_2Cl_2$, 25°C
d. N-Bromosuccinimide
e. $R^{22}COCl$ or $R^{22}CO-Im$ or other acylating agents.

The compounds bearing $R^1$ as $-SO_2NHR^y$ (where $R^y$ is heteroaryl or $R^9$) may be prepared by reacting the aromatic sulfonyl chloride 26 with appropriate heteroaryl amines as outlined in Scheme 8 to give 27. The sulfonyl chloride 26 may be prepared using similar chemistry to that outlined above. The sulfonyl chloride 26 may be the preferred intermediate for the synthesis of this class of compounds. The aromatic sulfonyl chlorides may also be prepared by reacting the sodium salt of aromatic sulfonic acids with $PCl_5$ or $POCl_3$ [C.M. Suter, The Organic Chemistry of Sulfur, John Wiley & Sons, 459, (1944)]. The aromatic sulfonic acid precursors may be prepared by chlorosulfonation of the aromatic ring with chlorosulfonic acid [E.H. Huntress and F.H. Carten, J. Amer. Chem. Soc., 62, 511 (1940)].

SCHEME 8

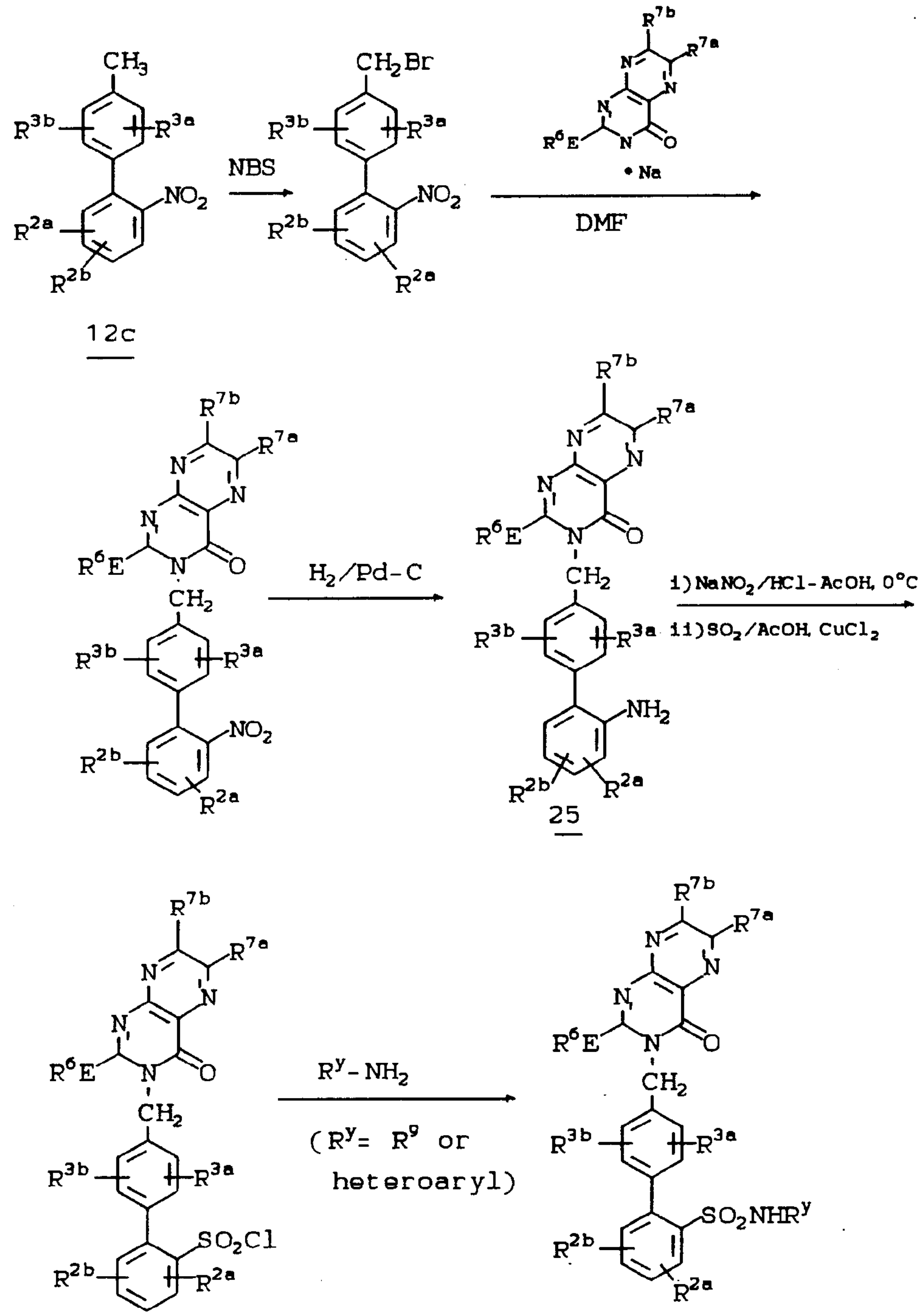


12c

25

26

27

The biaryl sulfonamides 28 and 29 (described in Scheme 7 as 21) can be prepared alternatively using palladium(0) catalyzed cross-coupling reactions of appropriate aryl-organotin precursors [J.K. Stille, Pure Appl. Chem., 57, 1771 (1985); T.R. Baiely, Tetrahedron Lett., 27, 4407 (1986); D.A. Widdowson and Y.Z. Zhang, Tetrahedron, 42, 2111 (1986)], as outlined in Scheme 9. The organotin compound 30 [S.M. Moerlein, J. Organometallic Chem., 319, 29 (1987)], obtained from the aromatic precursor 31, may be coupled with aryl sulfonamide 32 and 33 using $Pd(PPh_3)_4$ or $(PPh_3)_2PdCl_2$ as catalysts to give biaryl sulfonamide 28 and 29. Similarly, the benzyl bromide 34 may be alternatively prepared from the appropriate organotin precursor 35 using the Pd(0) catalyzed cross-coupling reaction as outlined in Scheme 12.

SCHEME 9

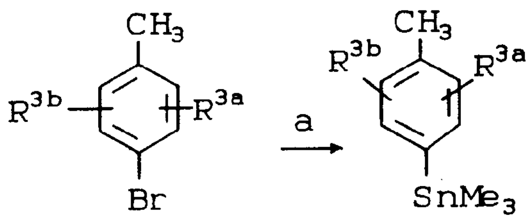


31     30

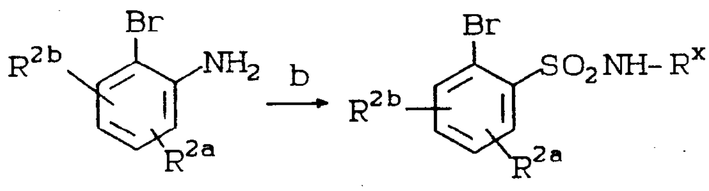


32 ($R^x$=-C($CH_3$)$_3$)

33 ($R^x$=-C($C_6H_5$)$_3$

30+(32 or 33) 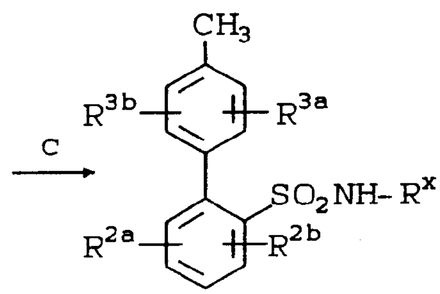


28 ($R^x$=-C($CH_3$)$_3$)

29 ($R^x$=-C($C_6H_5$)$_3$)

a. (i) t-BuLi/ether, -78°C
   (ii) $Me_3SnCl$

b. (i) $NaNO_2$/HCl
   (ii) $SO_2$, $CuCl_2$

c. $Pd(PPh_3)_4$, toluene, reflux or $(PPh_3)_2PdCl_2$, DMF, 90°C.

SCHEME 10

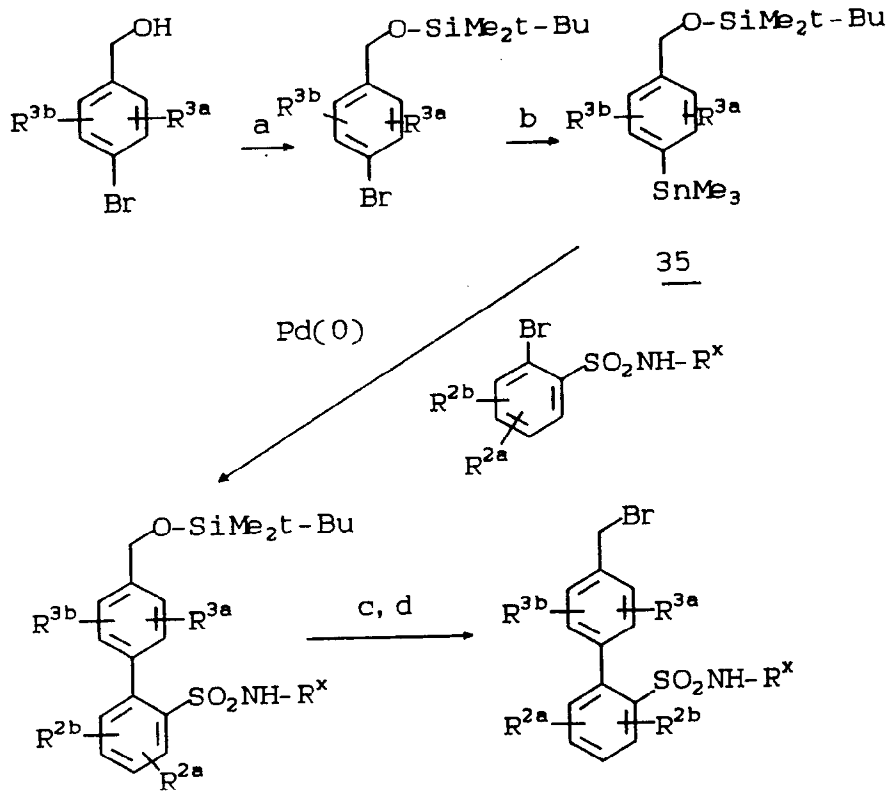


35

34(a) [$R^x$=-C($C_6H_5$)$_3$]

34(b) [$R^x$=-C($CH_3$)$_3$]

a. t-$BuMe_2Si$-Cl/imidazole, DMF

b. t-BuLi, -78°C, $Me_3SnCl$

c. tetrabutylammonium fluoride

d. $CBr_4$/$Ph_3P$.

The compounds bearing $R^1$= -$CH_2SO_2NHCOR^{22}$ and -$CH_2SO_2NHR^{22}$ may be prepared as outlined in Scheme 11. The key precursor aryl-methanesulfonyl chloride 36 may be prepared either from the reaction of arylmethylmagnesium chloride 37, obtained from the corresponding benzyl chloride 38 and magnesium, or by oxidation of the aryl-methylthioacetate 39 (prepared from the benzyl bromide 40 with chlorine in presence of trace amount of water) [Bagnay and Dransch, Chem. Ber., 93, 784 (1960)]. Alternatively, the aryl-methylthioacetate 39 can be oxidized with sulfuryl chloride in presence of acetic anhydride to form arylmethylsulfinyl chloride [S. Thea and G. Cevasco, Tet. Lett., 28, 5193 (1987)], which can be further oxidized with appropriate oxidizing agents to give the sulfonyl chloride 36. The compounds 41 and 42 can be obtained by

reacting the sulfonyl chloride 36 with appropriate amines.

## SCHEME 11

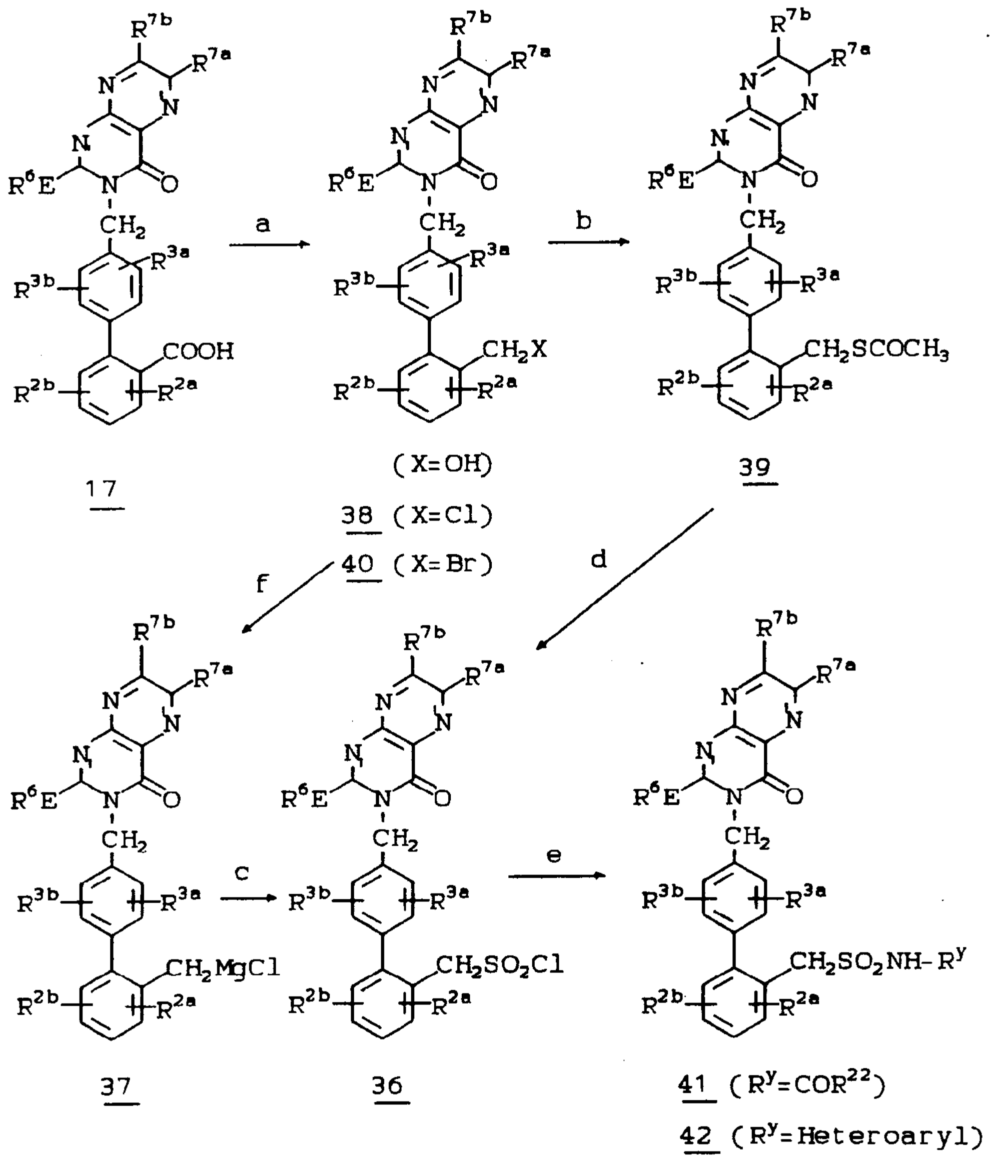


17

(X=OH)
38 (X=Cl)
40 (X=Br)

39

37

36

41 ($R^y$=$COR^{22}$)
42 ($R^y$=Heteroaryl)

SCHEME 11 (CONT'D)

a. (i) $EtOCOCl/Et_3N$, THF, 0°C
(ii) $NaBH_4$
(iii) $CCl_4$ or $CBr_4/PPh_3$
b. AcSK
c. $SO_2Cl_2$
d. $Cl_2$, AcOH, $H_2O$ or,
(i) $SO_2Cl_2$
(ii) oxidation
e. $R^yNH_2$ or,
(i) $NH_3$
(ii) acylation.

Compounds where $R^1$= $-NHSO_2NHR^{22}$ may be prepared by the reaction of appropriate primary amines with the sulfamide 43 [S.D. McDermott and W.J. Spillane, Synthesis, 192 (1983)], as described in Scheme 12. The compound 43 may be obtained from the corresponding N-t-butylsulfamide 44 after treatment with anhydrous trifluoroacetic acid [J.D. Catt and W.L. Matier, J. Org. Chem., 39, 566 (1974)]. The N-t-butylsulfamide 44 may be prepared by the reaction of the aromatic amine 45 (prepared as in Scheme 8) with t-butylsulfamoyl chloride [W.L. Matier, W.T. Comer and D. Deitchman, J. Med. Chem., 15, 538 (1972)].

SCHEME 12

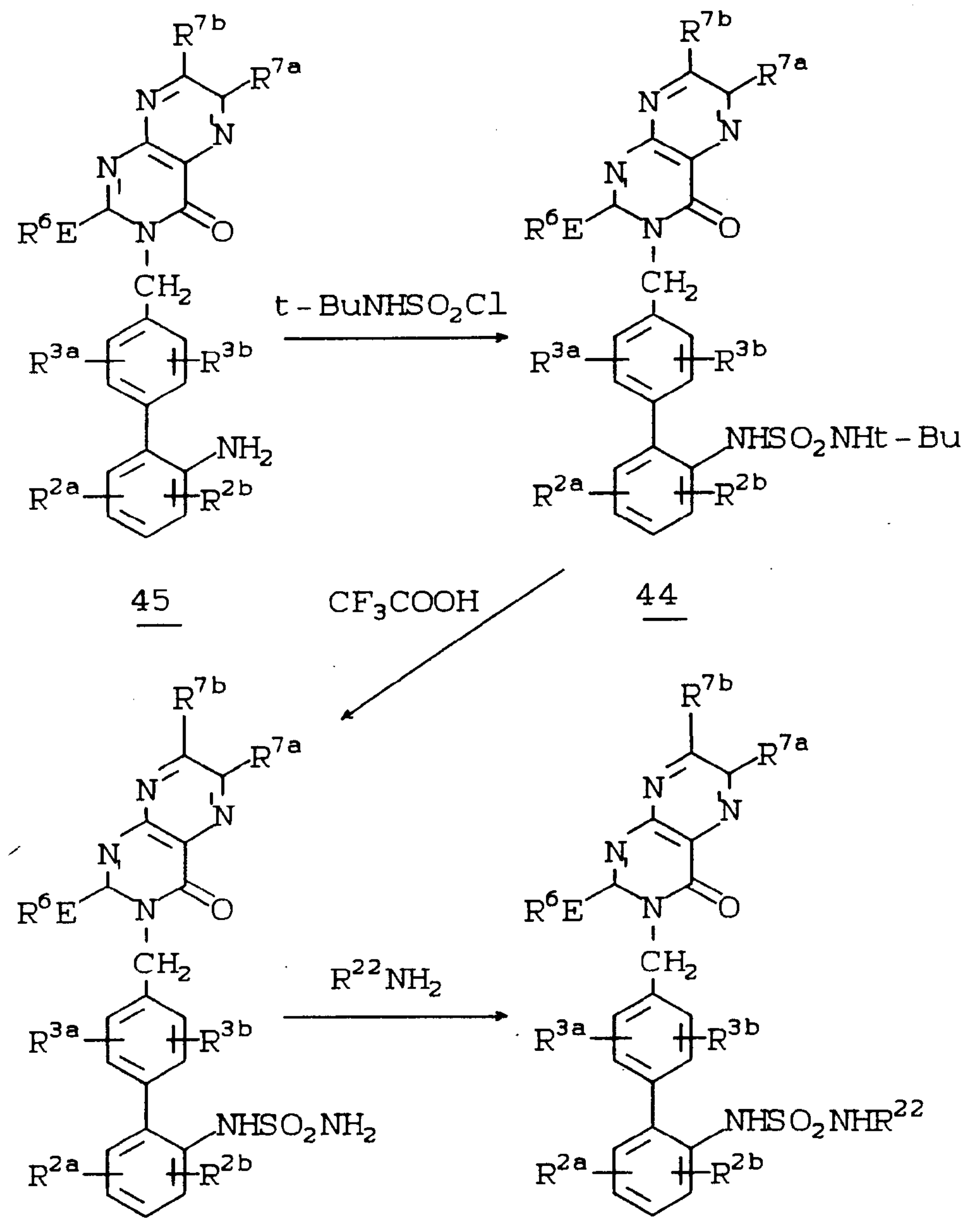


45 44 43

6 and 7 halo-pyrazino[2,3-d]pyrimidinones are very useful substrates for the introduction of substituents at the 6 or the 7 position. The halides of the fully alkylated precursor 1 may be displaced with a variety of cyclic and acyclic primary or secondary amines, thiois, alkoxides, enolates and amide anions and other similar nucleophiles.[4] The products can then be appropriately deprotected or modified into compounds of Formula I. Similarly, the 6 or 7 halo pyrazino[2,3-d]pyrimidinone 2 itself may be derivatized to give 6 or 7 substituted pyrazino[2,3-d]pyrimidinones of structure 2 where the $R^{7a}$ and $R^{7b}$ has been changed. The substituted pyrazino [2,3-d]pyrimidinone may then be alkylated in the manner of Scheme 1 to give compounds of Formula I.

[4] Jones, J.H.; Buckling, J.B.; Crogoe, E.J J. Med. Chem. 1967, 10. 899.

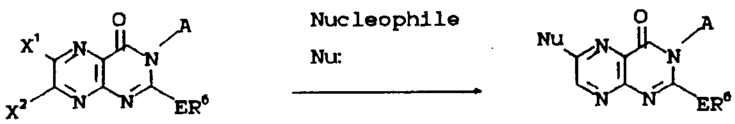


1) X = Cl, Br, I, H

product if $X^1$ = Cl, Br, I
$X^2$ = H
or

A = H or 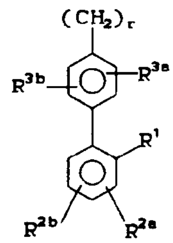

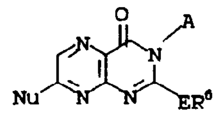

product if $X^1$ = H
$X^2$ = Cl, Br, I

It will be appreciated by those skilled in the art that functional group transformations can be conducted on aryl and heterocyclic rings to afford desired analogs. For example, esters may be converted to amides by heating them with amines and an amide nitrogen if present in the heterocycle may be alkylated using bases such as sodium hydride in DMF with the appropriate alkyl halide. Functional group protection throughout these syntheses will be chosen to be compatible with subsequent reaction conditions. Ultimately such protecting groups will be removed to generate the desired optimally active compounds of Formula I. For example, $R^1$ as carboxyl is often protected as its t-butyl ester which in the last step is removed by treatment with trifluoroacetic acid. Aqueous acetic acid at room temperature overnight is a preferred method to remove a trityl protecting group to liberate an $R^1$ tetrazole group.

The compounds of this invention form salts with various inorganic and organic acids and bases which are also within the scope of the invention. Such salts include ammonium salts, alkai metal salts like sodium and potassium salts, alkaline earth metal salts like the calcium and magnesium salts, salts with organic bases; e.g., dicyclohexylamine salts, N-methyl-D-glucamine, salts with amino acids like arginine, lysine, and the like. Also, salts with organic and inorganic acids may be prepared; e.g., HCl, HBr, $H_2SO_4$, $H_3PO_4$, methane-sulfonic, toluensulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Angiotensin II (AII) is a powerful arterial vasoconstrictor, and it exerts its action by interacting with specific receptors present on cell membranes. The compounds described in the present invention act as competitive antagonists of AII at the receptors. In order to identify AII antagonists and determine their efficacy in vitro, the following two ligand-receptor binding assays were established.

Receptor binding assay using rabbit aortae membrane preparation:

Three frozen rabbit aortae (obtained from Pel-Freeze Biologicals) were suspended in 5mM Tris -0.25M Sucrose, pH 7.4 buffer (50 ml) homogenized, and then centifuged. The mixture was filtered through a cheesecloth and the supernatant was centrifuged for 30 minutes at 20,000 rpm at 4°C. The pellet thus obtained was resuspended in 30 ml of 50mM Tris-5 mM $MgCl_2$ buffer containing 0.2% Bovine Serum Albumin and 0.2 mg/ml Bacitracin and the suspension was used for 100 assay tubes. Samples tested for screening were done in duplicate. To the membrane preparation (0.25 ml) there was added $^{125}$I-Sar$^1$Ile$^8$-angiotensin II [obtained from New England Nuclear] (10μl; 20,000 cpm) with or without the test sample and the mixture was incubated at 37°C for 90 minutes. The mixture was then diluted with ice-cold 50mM Tris-0.9% NaCl, pH 7.4 (4ml) and filtered through a glass fiber filter (GF/B Whatman 2.4" diameter). The filter was soaked in scintillation cocktail (10 ml) and counted for radioactivity using packard 2660 Tricarb liquid scintillation counter. The inhibitory concentration ($IC_{50}$) of potential AII antagonist which gives 50% displacement of the total specifically bound $^{125}$I-Sar$^1$Ile$^8$-angiotensin II was presented as a measure of the efficacy of such compounds as AII antagonists.

Receptor assay using Bovine adrenal cortex preparation

Bovine adrenal cortex was selected as the source of AII receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added 3H-angiotensin II (50mM) (10μl) with or without the test sample and the mixture was incubated at 37°C for 1 hour. The mixture was then diluted with Tris buffer (4ml) and filtered through a glass fiber filter (GF/B Whatman 2.4″ diameter). The filter was soaked in scintillation cocktail (10ml) and counted for radioactivity using Packard 2660 Tricarb liquid scintillation counter. The inhibitory concentration ($IC_{50}$) of potential AII antagonist which gives 50% displacement of the total specifically bound $^3H$-angiotensin II was presented as a measure of the efficacy of such compounds as AII antagonists.

Using the methodology described above, representative compounds of the invention were evaluated and were found to exhibit an activity of at least $IC_{50}$<50μM thereby demonstrating and confirming the utility of the compounds of the invention as effective AII antagonists.

The potential antihypertensive effects of the compounds described in the present invention may be evaluated using the methodology described below:

Male Charles River Sprague-Dawley rats (300-375 gm) were anesthetized with methohexital (Brevital; 50 mg/kg i.p.) and the trachea was cannulated with PE 205 tubing. A stainless steel pithing rod (1.5 mm thick, 150 mm long) was inserted into the orbit of the right eye and down the spinal column. The rats were immediately placed on a Harvard Rodent Ventilator (rate - 60 strokes per minute, volumn - 1.1 cc per 100 grams body weight). The right carotid artery was ligated, both left and right vagal nerves were cut, and the left carotid artery was cannulated with PE 50 tubing for drug administration, and body temperature was maintained at 37°C by a thermostatically controlled heating pad which received input from a rectal temperature probe. Atropine (1 mg/kg i.v.) was then administered, and 15 minutes later propranolol (1 mg/kg i.v.). Thirty minutes later, antagonists of formula (I) were administered intravenously or orally. Angiotensin II was then typically given at 5, 10, 15, 30, 45 and 60 minute intervals and every half hour thereafter for as long as the test compound showed activity. The change in the mean arterial blood pressure was recorded for each angiotensin II challenge and the percent inhibition of angiotensin II response was calculated.

Using the methodology described above, representative compounds of the invention were evaluated and all were found to exhibit an activity of at least $IC_{50}$<50μM thereby demonstrating and confirming the utility of the compounds of the invention as effective A II antagonists.

Thus, the compounds of the invention are useful in treating hypertension. They are also of value in the management of acute and chronic congestive heart failure and angina. These compounds are also expected to be useful in primary and secondary hyperaldosteronism, renal diseases such as diabetic nephropathy, glomerulonephritis, glomerular sclerosis, nephrotic syndrome, hypertensive nephrosclerosis, end stage renal disease, renal transplant therapy, renovascular hypertension, scleroderma, left ventricular dysfunction, systolic and diastolic dysfunction diabetic retinopathy, in the management of vascular disorders such as migraine or Raynaud's disease, as prophylaxis to minimize the atherosclerotic process, in neointimal hyperplasia follwoing angioplasty or vascular injury and to retard the onset of type II diabetes. The application of the compounds of this invention for these and similar disorders will be apparent to those skilled in the art.

The compounds of this invention are also useful to treat elevated intraocular pressure and to enhance retinal blood flow and can be administered to patients in need of such treatment with typical pharmaceutical formulations such as tablets, capsules, injectables and the like as well as topical ocular formulations in the form of solutions, ointments, inserts, gels, and the like. Pharmaceutical formulations prepared to treat intraocular pressure would typically contain about 0.1% to 15% by weight, preferably 0.5% to 2" by weight, of a compound of this invention. For this use, the compounds of this invention may also be used in combination with other medications for the treatment of glaucoma including choline esterase inhibitors such as physostigmine salicylate or demecarium bromide, parasympathomimetic agents such as pilocarpine nitrate, β-adrenergic antagonists such as timolol maleate, adrenergic agonists such as epinephrine and carbonic anhydrase inhibitors such as MK-507.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being

followed by a patient, concurrent medication, and other factors which those skilled in the art will recognize, the dosage range will generally be about 1 to 1000 mg. per patient per day which can be administered in single or multiple doses. Perferably, the dosage range will be about 5.0 to 500 mg. per patient per day; more preferably about 5 to 300 mg. per patient per day.

The compounds of this invention can also be administered in combination with other antihypertensives and/or diuretics. For example, the compounds of this invention can be given in combination with diuretics such as hydrochlorothiazide, chlorothiazide, chlorthalidone, methyclothiazide, furosemide, ethacrynic acid, triamterene, amiloride, atriopeptin and spironolactone; calcium channel blockers, such as diltiazem, felodipine, nifedipine, amlodipine, nimodipine, isradipine, nitrendipine and verapamil; β-adrenergic antagonists such as timolol, atenolol, metoprolol, propanolol, nadolol and pindolol; angiotensin converting enzyme inhibitors such as enalapril, lisinopril, captopril, ramipril, quinapril and zofenopril; renin inhibitors such as A-69729 and FK 906 and FK 744; α-adrenergic antagonists such as prazosin, doxazosin, and terazosin; sympatholytic agents such as methyldopa, clonidine and guanabenz, atriopeptidase inhibitors (alone or with ANP) such as UK-79300; serotonin antagonists such as ketanserin; $A_2$-adenosine receptor agonists such as CGS 22492C; potassium channel agonists such as pinacidil and cromakalim; and various other antihypertensive drugs including reserpine, minoxidil, guanethidine, hydralazine hydrochloride and sodium nitroprusside as well as combinations of the above-named drugs.

Combinations useful In the management of congestive heart failure include, in addition, compounds of this invention with cardiac stimulants such as dobutamine and xamoterol and phosphodiesterase inhibitors including amrinone and milrinone.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 5-500 milligrams per day range can be effectively combined at levels of the 1.0-500 milligrams per day range with the following compounds at the indicated per day dose range:
hydrochlorothiazide (6-100 mg), chlorothiazide (125-500 mg), ethacrynic acid (5-200 mg), amiloride (5-20 mg), furosemide (5-80 mg), propanolol (10-480 mg), timolol maleate (1-20 mg), methyldopa (125-2000 mg), felodipine (1-20 mg), nifedipine (5-120 mg), nitrendipine (5-60 mg) and diltiazem (30-540 mg). In addition, triple drug combinations of hydrochlorothiazide (5-100 mg) plus amiloride (5-20 mg) plus an angiotensin II antagonist of this invention (1-500 mg) or hydrochlorothiazide (5-100 mg) plus timolol maleate (5-60) plus an angiotensin II antagonist of this invention (1-500 mg) or hydrochlorothiazide (5-200 mg) and nifedipine (5-60 mg) plus an angiotensin II antagonist of this invention (1-500 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The following examples illustrate the preparation of the compounds of formula (I) and their incorporation into pharmaceutical compositions and as such are not to be considered as limiting the invention set forth in the claims appended hereto.

PREPARATION OF BIPHENYL SYNTHETIC INTERMEDIATES:

2-t-Butoxycarbonyl-4'-methylbiphenyl

To a solution of p-bromotoluene (30g) in dry ether (150 ml) at -78°C, a solution of t-BuLi in pentane (1.7M) (210 ml) was added slowly over a period of 1.5 hours, using a dropping funnel. The bath was then removed and the mixture was stirred at room temperature for an additional 2 hours. The content of the flask was then added slowly (using a cannula) at room temperature to a premixed solution of $ZnCl_2$ in ether (1M, 180 ml) and dry THF (360 ml). The mixture was stirred for 2 hours at that temperature and then the slurry was added (using a cannula) to a solution of 2-t-butoxycarbonyl iodobenzene (35.6 g) and $NiCl_2(Ph_3P)_2$ (2.1 g) in dry THF (360 ml). The mixture, after stirring at room temperature overnight (18 hours), was poured slowly under stirring into ice-cold 0.5N HCl (1500 ml). The organic layer was separated, and the aqueous phase was extracted with ether (3 x 300 ml). The combined organic layer was washed with water, brine and then dried over $MgSO_4$. Removal of the solvent gave the crude product as an oil (32 g). The material was purified on a silica-gel flash column using ethyl acetate-hexane (1:12) to give the titled compound as an oil (24 g, 76%). $^1H$ NMR ($CDCl_3$): $\delta$ 1.24 (s, 9H), 2.42,(s, 3H), 7.2-7.8 (m, 8H): FAB-MS: m/e 269(M+H).

4-Bromomethyl-2'-t-butoxycarboxylbiphenyl

To a solution of 2-t-butoxycarbonyl-4'-methylbiphenyl (25.3 g, 95 mmol) in $CCl_4$ (200 ml) were added freshly opened N-bromosuccinimide (17.6 g, 0.099 mole) and dibenzoyl peroxide (2.28 g, 0.0094 moles). The mixture was refluxed for 4 hours, cooled to room temperature and filtered. The filtrate was washed with sat. $NaHSO_3$ (1x50 ml), sat. $NaHCO_3$ (1x50 ml), water (1x50 ml), sat. NaCl (1x50 ml) and dried over $MgSO_4$. The solution was filtered, and concentrated in vacuo. The residue was dissolved in 100 ml of hot hexane. Crystallization gradually took place as the solution cooled: The flask was finally cooled to -20°C and the precipitate recovered by filtration. The solid was washed with ice cold hexanes and dried in vacuo to give 27 g (88%) of a white solid. $^1H$-NMR ($CDCl_3$): 1.23 (s, 9H), 4.53 (s, 2H), 7.2-7.5 (m, 7H), 7.68 (d, 1H).

2-Cyano-4'-methylbiphenyl

To a solution of p-bromotoluene (30 g) in dry ether (150 ml) at -78°C, a solution of t-BuLi in pentane (1.7M) (210 ml) was added slowly over a period of 1.5 hours, using a dropping funnel. The bath was then removed and the mixture was stirred at room temperature for an additional 2 hours. The contents of the flask was then added slowly (using a cannula) at room temperature to a premixed solution of $ZnCl_2$ in ether (1M) (180 ml) and dry THF (360 ml). The mixture was stirred for 2 hours at that temperature and then the slurry was added (using a cannula) to a solution of 2-bromobenzonitrile (21.3 g) and $NiCl_2(Ph_3P)_2$ (2.1 g) in dry THF (300ml). The mixture, after stirring at room temperature overnight (18 hours), was poured slowly under stirring into ice-cold 1N HCl (1500 ml). The organic layer was separated, and the aqueous phase was extracted with ether (3 x 300 ml). The combined organic layer was washed with water, brine and then dried over $MgSO_4$. Removal of the solvent gave the crude product as a semisolid mass (34 g). The material was purified on a silica-gel flash column using ethyl acetate-hexane (1:12) to give the desired nitrile as a low-melting solid (28 g, 88%). $^1H$ -NMR ($CDCl_3$): 2.42 (s, 3H), 7.2-7.8 (m, 8H); FAB-MS: m/e 194 ($M^+$+1).

Trimethylstannyl azide

To a concentrated solution of $NaN_3$ (1.2 kg, 18.5 moles) in water (3 L), a solution of trimethyltin chloride (600 g, 3 moles) in dioxane (400 ml) was added in three portions under vigorous stirring. A precipitate formed instantaneously. The mixture, after stirring overnight at room temperature, was filtered. The residue was washed with water, and dried under suction and then in vacuo over $P_2O_5$. Yield 541 g (88%), mp 120-122°C.

5-[2-(4'-Methylbiphenyl)]tetrazole

To a solution of 2-cyano-4'-methylbiphenyl (390 g, 2.02 moles) in toluene (2.3 L) was added trimethyltin azide (525 g, 2.55 moles) at room temperature. The mixture was refluxed for 24 hours, cooled to room temperature, filtered, washed with toluene and sucked dry in a funnel. The precipitate was resuspended in toluene (3.5 L) and THF (250 mL) was added. Anhydrous HCl was bubbled in at a moderate rate at room temperature to give a clear solution (45 minutes). Addition of HCl gas was continued for another 20 minutes with stirring whereupon a white precipitate formed. The reaction mixture was stirred overnight. The solid product was filtered, washed with toluene followed with ether and then dried under vacuum. This produced 250 g (53% yield of the tetrazole. m.p. 152-154°C; $^1$H-NMR ($CDCl_3$): 2.40 (s, 3H), 7.19 (dd, 1H), 7.55 (m, 2H), 8.25 (dd, 1H).

N-Triphenylmethyl-5-[2-(4′-methylbiphenyl]teterazole

To a cloudy solution of 5-[2-(4′-methylbiphenyl)]tetrazole (250 g (1.06 mole) in $CH_2Cl_2$ (4 L) was added triphenylmethylchloride (310 g 1.11 mole) at room temperature. The reaction mixture was stirred and triethylamine (190 mL, 138 g, 1.36 mole) was added portionwise. After addition, the mixture was stirred at reflux for 90 minutes. The solution was cooled to room temperature, washed with water (2x1 L) and dried over $MgSO_4$, filtered through a silica gel plug and concentrated on the rotovap to a solid. This was crystallized from toluene to give the product as an off-white solid (425 g, 84%); m.p. 166-168°C; $^1$H-NMR ($CDCl_3$): 2.28 (s, 3H), 6.9-7.05 (m, 10H), 7.2-7.5 (m, 12H), 7.9 (dd, 1H).

N-Triphenylmethyl-5-[2-(4′-bromomethylbiphenyl)]tetrazole

To a solution of N-triphenylmethyl-5-[2-(4′-methylbiphenyl)]tetrazole (425 g, 0.89 moles) in $CCl_4$ (4.0 L) was added N-bromsuccinimide (159 g, 0.89 mole) and dibenzoyl peroxide (22 g, 0.089 moles). The mixture was refluxed for 2 hours, cooled to room temperature and filtered. The filtrate was concentrated in vacuo to give a thick oil. The addition of ether (2.0 L) to this oil resulted in a clear solution followed by crystallization, filtration gave a white solid (367 g, 74%), m.p. 137-139.5°C; $^1$H-NMR ($CDCl_3$): 4.38 (s, 2H), 6.9-8.0 (m, 23H).

EXAMPLE 1

6-Pentylamino-2-propyl-3-[(2′-tetrazol-5-yl)biphenyl-4-yl)methyl]pyrazino[2,3-d]pyrimidin-4(3H)-one

Step A: 3-(N-Butyroylamino)-6-bromopyrazine-2-carboxamide

To a solution of 7.81 g (36 mmol) of 3-amino-6-bromo-pyrazine-2-carboxamide (prepared as in Albert, A., Ohta, K. J. Chem. Soc. 1971, 3729) in 100 ml of dry pyridine was added 5.75 g (54 mmol) of butyryl chloride. The reaction mixture was stirred at 50°C overnight, concentrated in vacuo and the residue was suspended in 250 ml of $CH_2Cl_2$. The solid residue was recovered by filtration and the filtrate was washed with sat, $NaHCO_3$ solution (2 x 30 ml), water (1 x30 ml) and brine (1 x 50 ml). The solution was dried over $MgSO_4$, filtered and concentrated in vacuo. The two residues were recrystalized from hot MeOH to give a total of 6.46 g (22.5 mmol) of white crystals. 62% yield. m.p.: 232-233°C. $^1$ H-NMR ($CD_3OD$-250MHz): 1.01 (t, 3H, J=7.38 Hz), 1.75 (m, 2H), 2.54 (t, 2H, J=7.43Hz), 8.61 (s, 1H). Anal. ($C_9H_{11}N_4O_2Br$) C, H, N.

Step B: 6-Bromo-2-propyl-pyrazino[2,3-d]pyrimidin-4(3H)-one.

To 6.21 g (0.022 mmol) of 3-(N-butyroylamino)-6-bromopyrazine-2-carboxamide was added 44 ml of 1M KOH (0.044 mmol) solution in water. The mixture was stirred at 55°C until complete dissolution was achieved. The reaction mixture was cooled to room temperature, acidified with acetic aid and the residue recovered by filtration to give 5.00 g (85% yield) of a pale green solid. A small amount was recrystalised from EtOAc to give a white solid m.p.=202-204°C (decomposed). $^1$ H-NMR ($CDCl_3$-400MHz): 1.06 (t, 3H, J=7.36Hz), 1.96 (m, 2H), 2.88 (t, 2H, J=7.41Hz), 8.99 (s, 1H), 11.9 (bs, 1H). Anal. ($C_9H_9N_4OBr$) C, H, N.

Step C: 6-Bromo-2-propyl-3-[(2′-(N-triphenylmethyltetrazol-5-yl)biphen-4-yl)methyl]pyrazino-[2,3-d]pyrimidin-4(3H)-one

To a solution of 0.2 g (0.74 mmol) of 6-Bromo-2-propyl-pyrazino[2,3-d]pyrimidin-4(3H)-one in 1 ml of dry DMF at 0°C was added 0.82 ml (0.82 mmol) of a 1M solution of sodium hexamethyldisilazide in THF. The solution was stirred for 10 minutes at which time a solution of 0.45 g (0.82 mol) of N-triphenylmethyl-5-[2-(4′-bromomethylbiphenyl)]tetrazole was added. The reaction mixture was stirred over night, diluted with 20 ml EtOAc and washed with water (2x10 ml) and brine (1x10 ml) and dried over $MgSO_4$. The suspension was filtered and the filtrate was concentrated in vacuo and the residue was purified by flash chromatography eluting with 35% EtOAc/hexanes to give 0.175 g of a pale yellow foam. 32% yield. $^1$H-NMR ($CDCl_3$-400MHz): 0.93 (3H, t, J=7.36 Hz), 1.82 (m, 2H), 2.71 (t, 2H, J=7.8Hz), 5.30 (bs, 2H), 6.88-7.50 (m, 22H), 8.92 (m, 1H), 8.95 (s, 1H).

Step D: 6-Pentylamino-2-propyl-3-[(2′-tetrazol-5-yl)biphen-4-yl)methyl]pyrazino[2,3-d]pyrimidin-4(3H)-one

A solution of 0.056 (0.07 mmol) of 6-bromo-2-propyl-3-[(2′-(N-triphenylmethyl-tetrazol-5-yl)biphen4-yl)methyl]pyrazino(2,3-d]pyrimidin-4(3H)-one was heated with 20 mg (0.22 mmol) n-pentylamine in 1 ml of methoxyethanol at 100°C for 4 hours. The reaction mixture was concentrated in vacuo and purified the residue was purified by flash chromatography over silica gel eluting with a gradient of 85% EtOAc/14% hexanes/1% AcOH to 80%EtOAc/14% hexanes/ 5% MeOH/1% AcOH to give 20.4 mg of a pale yellow foam. 53% yield. $^1$H-NMR ($CDCl_3$-400MHz): 0.82-0.91 (m, 6H), 1.28 (bm, 4H), 1.59 (t, 2H, J=7.1Hz), 1.63 (m, 2H), 2.61 (t, 2H, J=7.32 Hz), 3.31 (bm, 2H), 5.20 (bs, 2H), 5.91 (bs, 1H), 6.95 ( s, 4H), 7.31 (d, 1H, J=7.3Hz), 7.49 (m, 2H), 7.81 (d. 1H, J=7.70Hz), 8.13 (s, 1H).

EXAMPLE 2

6-Bromo-2-propyl-3-[(2′-tetrazol-5-yl)biphen-4-yl)methyl]pyrazino[2,3-d]pyrimidin-4(3H)-one

0.119 g (0.16 mmol) of 6-bromo-2-propyl-3[(2′-(N-triphenylmethyl-tetrazol-5-yl)biphen-4-yl)methyl]pyrazino[2,3-d]pyrimidin-4(3H)-one was stirred with 2 ml of a mixture of 3:1:1 AcOH:water:THF overnight. The reaction mixture was concentrated in vacuo and the residue was purified by flash chromatography over silica gel eluting with 60% EtOAc/hexanes/1% AcOH to give 26.7 mg of a yellow glass. 31% yield. $^1$H-NMR ($CDCl_3$-400MHz):0.95 (t, 3H, J=7.3Hz), 1.82 (m, 2H), 2.79 (t, 2H, J=7.4 Hz), 5.34 (bs, 2H), 6.95-7.56 (m, 8H, 7.82 (bd, 1H, J=7.0Hz), 8.82 (bs, 1H).

EXAMPLE 3

6-Morpholino-2-propyl-3-[(2′-tetrazol-5-yl)-biphen-4-yl-methyl]pyrazino[2,3-d]pyrimidin-4(3H)-one

Step A: 6-Bromo-2-propyl-3-[(2′-cyanobiphen-4-yl)methyl]pyrazino[2,3-d]pyrimidin-4(3H)-one

To a suspension of 0.205 g (8.5 mmol) of NaH in 10 ml of dry DMF at 0°C was added 2.0 g (7.4 mmol) of 6-bromo-2-propyl-pyrazino[2,3-d]pyrimidin-4(3H)one to give a dark suspension. After 30 minutes a solution of 2.39 g (8.8 mmol) of 2-cyano-4′-bromomethylbiphenyl in DMF was added and the reaction mixture was allowed to warm to room temperature over night. The reaction mixture was diluted with 200 ml of EtOAc and washed with water (3x50 ml), brine (1x50 ml) and the organic phase was dried over $MgSO_4$. The suspension was filtered and concentrated in vacuo and the residue was purified by flash chromatography over silica gel eluting with 50% EtOAc/hexanes to give 2.72 g of an orange foam. 80% yield. $^1$H-NMR ($CDCl_3$-400MHz): 1.01 (t, 3H, J=7.5Hz), 1.90 (m, 2H), 2.85 (t, 3H, J=7.81 Hz), 5.48 (bs, 2H), 7.31-7.75 (m, 8H), 8.96 (s, 1H).

Step B: 6-Morpholino-2-propyl-3-[(2′-cyanobiphen-4-yl)methyl]pyrazino[2.3-d]pyrimidin-4(3H)-one

To a solution of 0.2 g (0.43 mmol) of 6-bromo-2-propyl-3-[(2′-cyanobiphen-4-yl)methyl]pyrazino[2,3-d]pyrimidin-4(3H)-one in 1 ml of dry DMF was added 0.189 g (2.17 mmol) of morpholine. The solution was heated at 75°C for 1 hour, diluted with 10 ml of EtOAc, filtered and the filtrate was concentrated in vacuo. The residue was purified by flash chromatography over silica gel eluting with 5% $MeOH/CH_2Cl_2$ to give 0.20 g (0.43 mmol) of a yellow powder. 100% yield. $^1$ H-NMR ($CDCl_3$-300MHz): 1.02 (t, 3H), 1.88 (m, 2H), 2.81 (t, 2H), 3.72-3.88 (m, 8H), 5.46 (bs, 2H), 7.31-7.77 (m, 8H), 8.67 (s, 1H).

Step C: 6-Morpholino-2-propyl-3-[(2′-tetrazol-5-yl)biphen-4-yl)methyl]pyrazino[2,3-d]pyrimidin-4(3H)-one

A mixture of 0.15 (0.32 mmol) of 6-morpholino-2-propyl-3-[(2′-cyanobiphen-4-yl)methyl]pyrazino[2,3-d]pyrimidin-4(3H)-one and 0.19 g (0.96 mmol) of trimethyl tin azide were heated at 110°C overnight. A further 0.19 g (0.96 mmol) of trimethyl tin azide was added and the mixture was heated at 120°C for 12 hours to achieve complete consumption of starting material. The mixture was diluted with 20 ml of EtOAc and washed with saturated $NH_4Cl$ (2x5 ml), water (1x10 ml) and brine (1x10 ml). The organic phase was dried over $MgSO_4$, filtered and concentrated in vacuo. The residue was purified by flash chromatography over silica gel eluting with 5% $MeOH/CH_2Cl_2$ to give 0.13 g of a yellow glass. $^1$H-NMR indicated that trimethyl tin azide was still present. The crude product was stirred in 10 ml of $CH_2Cl_2$ with 5 ml of IN HCl. The aqueous phase was extracted with $CH_2Cl_2$ (2x5 ml) but little of the product had dissolved in the aqueous phase. The organic phase was dried over $MgSO_4$, filtered and concentrated in vacuo and the residue was purified by MPLC over LH20 (3 cm x 180 cm column)

eluting with MeOH to give 0.078 g of a yellow glass. 48% yield. $^1$H-NMR ($CD_3OD$-400MHz): 0.97 (3H, t, J=7.0 Hz), 1.78 (m, 2H), 2.77 (t, 2H, J=7.5Hz), 3.80 (bs, 8H), 5.48 (bs, 2H), 7.17 and 7.11 (ABq, 4H, J=8.0 Hz), 7.55 (m, 2H), 7.67 (m, 2H), 8.82 (bs, 1H).

EXAMPLE

Typical Pharmaceutical Compositions Containing a Compound of the Invention

**A**: Dry Filled Capsules Containing 50 mg of Active Ingredient Per Capsule

| Ingredient | Amount per capsule (mg) |
|---|---|
| Active ingredient | 50 |
| Lactose | 149 |
| Magnesium stearate | 1 |
| Capsule (size No. 1) | 200 |

The active ingredient is reduced to a No. 60 powder and the lactose and magnesium stearate is then passed through a No. 60 blotting cloth onto the powder. The combined ingredients are mixed for about 10 minutes and filled into a No. 1 dry gelatin capsule.

**B**: Tablet

A typical tablet contains active ingredient (25 mg), pregelatinized starch USP (82 mg), microcrystalline cellulose (82 mg) and magnesium stearate (1 mg).

**C**: Combination Tablet

A typical combination tablet contains, for example, a diuretic such as hydrochlorothiazide (50 mg) active ingredient pregelatinized starch USP (82 mg), microcrystalline cellulose (82 mg) and magnesium stearate (1 mg).

**D**: Suppository

Typical suppository formulations for rectal administration contains active ingredient (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or a diuretic and/or an angiotensin converting enzyme and/or a calcium channel blocker in pharmaceutically effective amounts as described, for example, in C above.

**E**: Injection

A typical injectible formulation contains active ingredient, (10 mg), sodium phosphate dibasic anhydrous (11.4 mg) benzylalcohol (0.01 ml) and water for injection (1.0 ml). Such an injectible formulation can also include a pharmaceutically effective amount of another active ingredient such as another antihypertensive and/or a diuretic and/or an angiotensin converting enzyme inhibitor and/or a calcium channel blocker.

## Claims

1. A compound of structural formula I

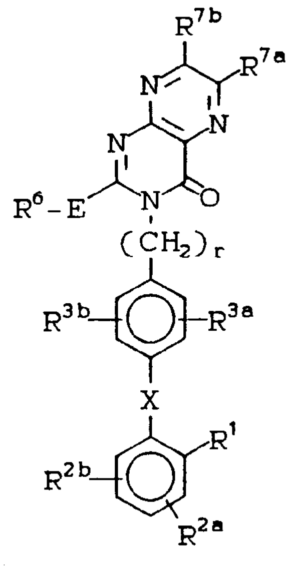

(I)

or a pharmaceutically acceptable salt thereof

wherein:

$R^1$ is

(a) $-CO_2R^4$,

(b) $-SO_3R^5$,

(c) $-NHSO_2CF_3$,

(d) $-PO(OR^5)_2$,

(e) $-SO_2-NH-R^9$,

(f) $-CONHOR^5$,

(g)

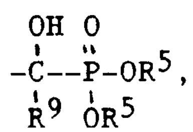

(h)

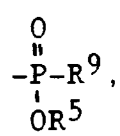

(i) $-SO_2NH$-heteroaryl,

(j) $-CH_2SO_2NH$-heteroaryl,

(k) $-SO_2NH-CO-R^{22}$,

(l) $-CH_2SO_2NH-CO-R^{22}$,

(m) $-CONH-SO_2R^{22}$,

(n) $-CH_2CONH-SO_2R^{22}$

(o) $-NHSO_2NH-CO-R^{22}$

(p) $-NHCONHSO_2-R^{22}$,

(q)

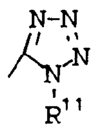 or 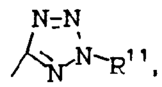

(r)

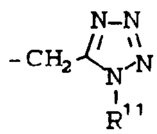 or 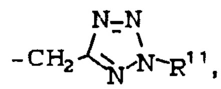

(s)

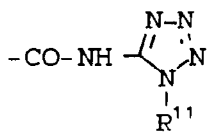 or 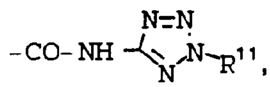

(t) $-CONHNHSO_2CF_3$ ,
(u) $-SO_2NH-CH$,
(v)

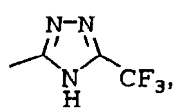

(w)

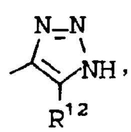

(x) $-PO(OR^5)(OR^4)$
(y) $-SO_2NHCONR^4R^{22}$,
$R^{2a}$ and $R^{2b}$ are each independently
(a) H,
(b) halo
(c) $NO_2$,
(d) $NH_2$,
(e) $C_1$-$C_4$-alkylamino,
(f) di($C_1$-$C_4$-alkyl)amino
(g) $SO_2NHR^9$,
(h) $CF_3$,
(i) $C_1$-$C_6$-alkyl,
(j) $C_1$-$C_6$-alkoxy,
(k) $C_1$-$C_6$-alkylthio,
(l) $C_2$-$C_6$-alkenyl,
(m) $C_2$-$C_6$-alkynyl;
(n) aryl,
(o) aryl($C_1$-$C_4$-alkyl), or

(p) $C_3$-$C_7$-cycloalkyl;

$R^{3a}$ is

(a) H,

(b) halo,

(c) $C_1$-$C_6$-alkyl,

(d) $C_1$-$C_6$-alkoxy, or

(e) $C_1$-$C_6$-alkoxy-$C_1$-$C_4$-alkyl;

$R^{3b}$ is

(a) H,

(b) halo,

(c) $NO_2$,

(d) $C_1$-$C_6$-alkyl,

(e) $C_1$-$C_6$-acyloxy,

(f) $C_3$-$C_7$-cycloalkyl,

(g) $C_1$-$C_6$-alkoxy,

(h) -$NHSO_2R^4$,

(i) hydroxy($C_1$-$C_4$-alkyl),

(j) aryl($C_1$-$C_4$-alkyl),

(k) $C_1$-$C_4$-alkylthio,

(l) $C_1$-$C_4$-alkylsulfinyl,

(m) $C_1$-$C_4$-alkylsulfonyl,

(n) $NH_2$,

(o) $C_1$-$C_4$-alkylamino,

(p) di($C_1$-$C_4$-alkyl)amino,

(q) polyfluoro-$C_1$-$C_4$-alkyl-,

(r) -$SO_2$-$NHR^9$,

(s) aryl as defined below,

(t) furyl,

(u) $C_2$-$C_6$-alkenyl, or

(v) $C_2$-$C_6$-alkynyl;

$R^4$ is H, aryl, heteroaryl or $C_1$-$C_6$ alkyl unsubstituted or substituted with aryl or heteroaryl;

$R^{4a}$ is aryl, or $C_1$-$C_6$-alkyl either unsubstituted or optionally substituted with aryl;

$R^5$ is H,

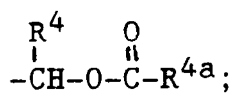

E is a single bond, -$NR^{13}(CH_2)_s$-$CH_3$, -$S(O)_x(CH_2)_x$-$CH_3$ where x is 0 to 2 and s is 0 to 5, -CH(OH)-, -O-, CO-;

$R^6$ is

(a) aryl either unsubstituted or substituted with 1 or 2 substituents selected from the group consisting of halo, -0-$C_1$-$C_4$-alkyl, $C_1$-$C_4$-alkyl, -$NO_2$, -$CF_3$, -$SO_2NR^9R^{10}$ -S-$C_1$-$C_4$-alkyl, -OH, -$NH_2$, $C_3$-$C_7$-cycloalkyl, and $C_3$-$C_{10}$-alkenyl;

(b) $C_1$-$C_6$-alkyl, $C_2$-$C_5$-alkenyl or $C_2$-$C_5$-alkynyl each of which can be substituted with a substituent selected from the group consisting of aryl, $C_3$-$C_7$-cycloalkyl, halo, -$CF_3$, -$CF_2CF_3$, -$NH_2$, -NH($C_1$-$C_4$-alkyl), -$OR^4$, -N($C_1$-$C_4$-alkyl)$_2$, -NH-$SO_2R^4$, -$COOR^4$, and -$SO_2NHR^9$;

(c) heteroaryl

(d) $C_3$-$C_7$-cycloalkyl;

(e) perfluoro-$C_1$-$C_4$-alkyl, or

(f) H;

$R^{7a}$ and $R^{7b}$ are independently

(a) H,

(b) $C_1$-$C_8$-alkyl unsubstituted or substituted with a substituent selected from the group consisting of -guanidino, $COOR^4$, -$CON(R^4)_2$, -O-$COR^4$, -aryl, -heteroaryl, -tetrazol-5-yl, -$CONHSO_2R^{22}$, -$SO_2NH$-, heteroaryl, -$SO_2NHCOR^{22}$, -$PO(OR^4)_2$, -$PO(OR^4)R^9$, -$SO_2NH$-CN, and -$NR^{10}COOR^{22}$,

(c) -CO-aryl,

(d) $-C_3-C_7$-cycloalkyl,
(e) $-C_1-C_4$-perfluoroalkyl,
(f) $-COOR^4$,
(g) $-SO_3H$,
(h) $-NR^4R^{22}$,
(i) $-NR^4COR^{22}$,
(j) $-NR^4COOR^{22}$,
(k) $-NO_2$,
(l) $-N(R^4)SO_2R^{22}$,
(m) $-NR^4CONR^4R^{22}$,
(n)

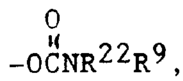

(o) -aryl or -heteroaryl,,
(p) $-NHSO_2CF_3$,
(q) $-SO_2NH$-heteroaryl,
(r) $-SO_2NHCOR^{22}$,
(s) $-CONHSO_2R^{22}$,
(t) $-PO(OR^4)2$,
(u) $-PO(OR^4)R^4$,
(v) $-COR^4$,
(w) $-NR^4SO_2NR^4R^{22}$,
(x) $-NR^4SO_2OR^{22}$
(y) $-CONR^4R^{22}$,
(z)

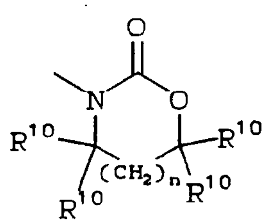

where n=0 or 1.
(aa)

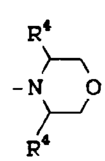

(bb)

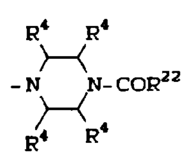

(cc)

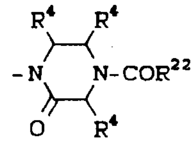

(dd)

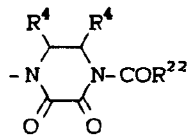

(ee)

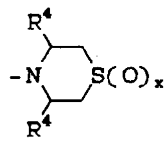

(ff)

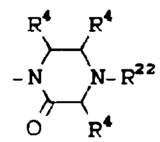

(gg)

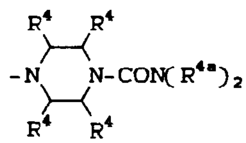

(hh)

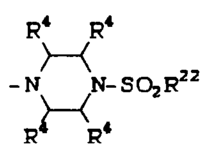

with the proviso that one of $R^{7a}$ and $R^{7b}$ is other than H;
$R^9$ is H, $C_1$-$C_5$-alkyl, aryl, heteroaryl or arylmethyl;
$R^{10}$ is H, $C_1$-$C_4$-alkyl;
$R^{11}$ is H, $C_1$-$C_6$-alkyl, $C_2$-$C_4$-alkenyl, $C_1$-$C_4$-alkoxy -$C_1$-$C_4$- alkyl, or

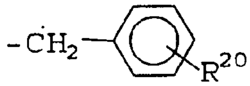

$R^{12}$ is -CN, $-NO_2$ or $-CO_2R^4$;
$R^{13}$ is H, ($C_1$-$C_4$-alkyl)CO-, $C_1$-$C_6$-alkyl, allyl, $C_3$-$C_6$-cycloalkyl, aryl or arylmethyl;
$R^{14}$ is H, $C_1$-$C_8$-alkyl, $C_1$-$C_8$-perfluoroalkyl, $C_3$-$C_6$-cycloalkyl, aryl or arylmethyl;
$R^{15}$ is H, $C_1$-$C_6$-alkyl;
$R^{16}$ is H, $C_1$-$C_6$-alkyl, $C_3$-$C_6$-cycloalkyl, aryl or arylmethyl;
$R^{17}$ is $-NR^9R^{10}$, $-OR^{10}$, $-NHCONH_2$, $-NHCSNH_2$,

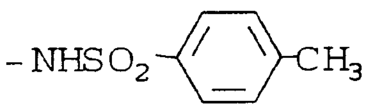 or 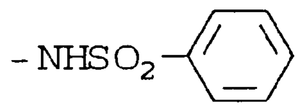 ;

$R^{18}$ and $R^{19}$ are independently $C_1$-$C_4$-alkyl or taken together are $-(CH_2)_q-$ where q is 2 or 3;
$R^{20}$ is H, $-NO_2$, $-NH_2$, -OH or $-OCH_3$;
$R^{22}$ is
(a) aryl,
(b) heteroaryl,
(c) $C_3$-$C_7$-cycloalkyl,
(d) $C_1$-$C_6$-alkyl unsubstituted or substituted with a substituent selected from the group consisting of aryl, heteroaryl, -OH, -SH, $C_1$-$C_4$-alkyl, -O($C_1$-$C_4$-alkyl),-S($C_1$-$C_4$-alkyl), $-CF_3$, halo, $-NO_2$, $-CO_2H$, $CO_2$-($C_1$-$C_4$-alkyl), $-NH_2$, -NH($C_1$-$C_4$-alkyl), $-N(C_1\text{-}C_4\ \text{alkyl})_2$, $-PO_3H_2$, -PO(OH)(O-$C_1$-$C_4$-alkyl), $-PO(OR^4)R^9$;
(e) perfluoro-$C_1$-$C_4$-alkyl;
X is
(a) a carbon-carbon single bond,
(b) -CO-,
(c) -O-,
(d) -S-,
(e)

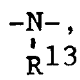

(f)

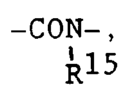

(g)

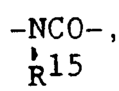

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,

(p) -CH=CH-,
(q) -CF=CF-,
(r) -CH=CF-,
(s) -CF=CH-,
(t) $-CH_2CH_2-$,
(u) $-CF_2CF_2-$,
(v)

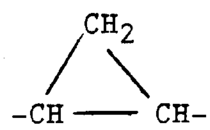
or
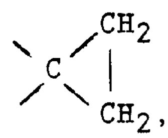

(w)

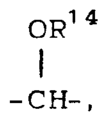

(x)

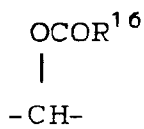

(y)

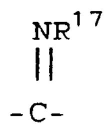
,

or
(z)

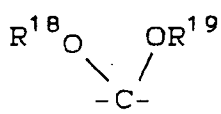
;

and
r is 1 or 2;

**2.** The compound of Claim 1 wherein;
$R^1$ is
(a) -COOH,

(b)

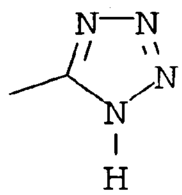,

(c) $-NH-SO_2CF_3$;
(d) $-SO_2NH$-heteroaryl,
(e) $-CH_2SO_2NH$-heteroaryl,
(f) $-SO_2NH-CO-R^{22}$,
(g) $-CH_2SO_2NH-CO-R^{22}$,
(h) $-CONH-SO_2R^{22}$,
(i) $-CH_2CONH-SO_2R^{22}$,
(j) $-NHSO_2NHCO-R^{22}$,
(k) $-NHCONHSO_2-R^{22}$,

$R^{2a}$ is H;

$R^{2b}$ is H, F, Cl, $CF_3$, $C_1$-$C_6$-alkyl, $C_2$-$C_6$-alkenyl, $C_2$-$C_6$-alkynyl, or aryl;

$R^{3a}$ is H;

$R^{3b}$ is H, F, CL, $CF_3$, $C_1$-$C_4$-alkyl, $C_2$-$C_4$-alkenyl, $C_2$-$C_4$-alkynyl, $C_5$-$C_6$-cycloalkyl, $-COOCH_3$, $-COOC_2H_5$, $-SO_2-CH_3$, $NH_2$, $-N(C_1-C_4\text{-alkyl})_2$ or $-NH-SO_2CH_3$;

E is a single bond, -O- or -S-;

$R^6$ is

(a) $C_1$-$C_5$ alkyl unsubstituted or substituted with a substituent selected from the group consisting of $C_3$-$C_5$-cycloalkyl, Cl, $CF_3$, $CCl_3$, $-O-CH_3$, $-OC_2H_5$, $-S-CH_3$, $-S-C_2H_5$, phenyl, or F;
(b) $C_2$-$C_5$-alkenyl or $C_2$-$C_5$-alkynyl; or,
(c) $C_3$-$C_5$-cycloalkyl;

$R^{7a}$ and $R^{7b}$ are independently

(a) H,
(b) $C_1$-$C_8$-alkyl unsubstituted or substituted with COOR, $OCOR^{4a}$, OH, aryl, or $-(CH_2)_{1-4}R^4$;
(c) $-NO_2$,
$R^4O$
(d) $-N-C-R^{22}$,
(e) $-CONR^4R^{22}$,
(f)

O
$-NR^4-C-O-R^{22}$,

(g) $-NR^4R^{22}$,
(h) halo,
(i) $-CF_3$,
(j) $-CO_2R^4$,
(k) -CO-aryl as defined above,
(l) $-N(R^4)SO_2R^{22}$,
(m) aryl or heteroaryl as defined above,
(n) $-NR^4CONR^4R^{22}$,
(o) $-N(R^4)SO_2N(R^4)R^{22}$;
(p)

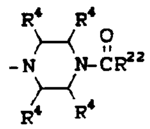

(q)

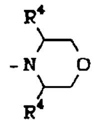

with the proviso that one of $R^{7a}$ and $R^{7b}$ is other than H;
X is a single bond;
r is one.

3. The compound of Claim 2 wherein:
$R^1$ is
(a) -COOH,
(b)

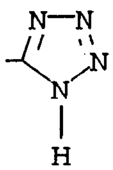

(c) $-NH-SO_2-CF_3$,
(d) $-SO_2NH$-heteroaryl as defined above.
(e) $-SO_2NH-CO-R^{22}$,
(f) $-CONH-SO_2R^{22}$.
E is a single bond;
r is one,
$R^{2a}$, $R^{2b}$, $R^{3a}$ and $R^{3b}$ are each H, $-C_1-C_6$-alkyl, $-C_2-C_6$-alkenyl, $-C_2-C_6$-alkynyl, -Cl, -F, $-NO_2$, $-CF_3$;
$R^6$ is $-C_1-C_4$-alkyl, -cyclopropyl, $-CH_2CH_2CH_2CF_3$, $-CH_2CH_2CF_3$, $-C_2-C_5$-alkenyl, -cyclopropylmethyl.
$R^{7a}$ and $R^{7b}$ are each independently H, $-C_1-C_4$-alkyl, $-NO_2$, $-NR^4R^{22}$, $-NR^4COOR^{22}$, $-CH_2COOR^{4a}$, $-S(O)_x-R^{22}$ alkyl, $NR^4CONR^4R^{22}$, $CH_2OCO(C_1-C_4$- alkyl), $NR^4COR^{22}$, $CO_2R^4$, -F, $-CH_2Ph$, $-CONR^4R^{22}$.

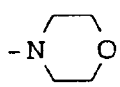 , 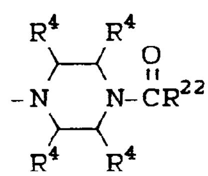

with the proviso that one of $R^{7a}$ and $R^{7b}$ is other than H;

4. The compound of Claim 3 wherein:
$R^1$ is
(a) COOH,

(b)

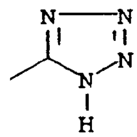

(c) $-SO_2NHCOR^{22}$,
(d) $-CONHSO_2R^{22}$,
(e) $-NHSO_2CF_3$;

$R^{2a}$, $R^{2b}$, $R^{3a}$ and $R^{3b}$ are each H, $-C_1-C_4$-alkyl, -Cl or F;

$R^{7a}$ or $R^{7b}$ are: H, halo, $NR^4R^{22}$, $NR^4COR^{22}$, $C_1-C_4$alkyl, $NR^4COOR^{22}$, $NR^4CONR^4R^{22}$

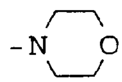 , 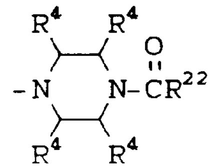

5. The compound of Claim 4 which is

(1) 2-n-Butyl-3-[(2′-carboxybiphen-4-yl)methyl]pyrazino[2,3-d]pyrimidin-4(3H)-one;
(2) 2-n-Butyl-3-[(2′-(tetrazol-5-yl)biphen-4-yl)methyl]pyrazino[2,3-d]pyrimidin-4(3H)-one;
(3) 2-n-Butyl-3-[2′-(carboxybiphen-4-yl)methyl]pyrazino[3,2-d]pyrimidin-4(3H)-one;
(4) 2-n-Butyl-3-[(2′-(tetrazol-5-yl)biphen-4-yl)methyl]pyrazino[4,3-d]pyrimidin-4(3H)-one;
(5) 2-n-Butyl-7-isopropyl-3-[(2′-(tetrazol-5-yl)biphen-4-yl)methyl]pyrazino[4,3-d]pyrimidin-4-(3H)-one;
(6) 6-Amino-2-n-Butyl-3-[(2′-(tetrazol-5-yl)biphen4-yl)methyl]pyrazino[2,3-d]pyrimidin-4(3H)-one;
(7) 6-Acetamido-2-n-Butyl-3-[(2′-(tetrazol-5-yl)biphen-4-yl)methyl]pyrazino[2,3-d]pyrimidin-4-(3H)-one;
(8) 2-n-Butyl-6-methyl-3-[(2′-(tetrazol-5-yl)biphen4-yl)methyl]pyrazino[3,4-d]pyrimidin-4(3H)-one;
(9) 2-n-Butyl-3-[(2′-(tetrazol-5-yl)biphen-4-yl) methyl]-6-thiomethylpyrazino[2,3-d]pyrimidin-4-(3H)-one;
(10) 2-n-Butyl-7-carboxy-3-[(2′-(tetrazol-5-yl)biphen-4-yl)methyl]pyrazino[2,3-d]pyrimidin-4-(3H)-one;
(11) 2-n-Butyl-7-(N-isopropylcarbamoyl)amino-3-[(2′-(tetrazol-5-yl)biphen-4-yl)methyl]pyrazino[3,2-d]pyrimidin-4(3H)-one;
(12) 2-n-Butyl-6-(N-isobutyloxycarbonyl)amino-3-[(2′-(tetrazol-5-yl)biphen-4-yl)methyljpyrazino[2,3-d]pyrimidin-4(3H)-one;
(13) 2-n-Butyl-6-[N-(morpholin-4-yl)carbamoyl)-N-methyl]amino-3-[(2′-tetrazol-5-yl)biphen-4-yl)methyl]pyrazino[2,3-d]pyrimidin,4(3H)-one;
(14) 2-n-Butyl-6-(N-isopropyloxycarbonyl-N-methyl)amino-3-[(2′-tetrazol-5-yl)biphen-4-yl)methyl]-pyrazino[2,3-d]pyrimidin-4(3H)-one;
(15) 6-(N-Benzyloxycarbonyl-N-methyl)amino-2-n-butyl-3-[(2′-(tetrazol-5-yl)biphen-4-yl)methyl]pyrazino[2,3-d]pyrimidin-4(3H)-one;
(16) 3-[(2′-(N-Benzoylsulfonamido)biphen-4-yl)]methyl-2-n-butyl-6-(N-isopropyloxycarbonyl-N-benzyl) aminopyrazino[2,3-d]pyrimidin-4(3H)-one;
(17) 2-n-Butyl-6-(N-isopropyloxycarbonyl-N-methyl)amino-3-[(2′-(N-trifluoromethylsulfonylcarboxamido)biphen-4-yl)methyl]pyrazino[2,3-d]-pyrimidin-4(3H)-one;
(18) 2-n-Butyl-3-[(2′-(tetrazol-5-yl)biphen-4-yl)methyl]pyrazino[3,2-d]pyrimidin-4(3H)-one;
(19) 6-[N-Benzyl-N-n-butyloxycarbonyl]amino-2-propyl-3-[(2′-(tetrazol-5-yl))biphen-4-yl) methyl]-pyrazino[2,3-d]pyrimidin-4(3H)-one;
(20) 2-n-Butyl-6-(N-methyl-N-isobutyloxycarbonyl) amino-3-[(2′-(tetrazol-5-yl)biphen-4-yl)methyl] pyrazino[3,2-d]pyrimidin-4(3H)-one;
(21) 6-(N-Benzyl-N-butanoyl)amino-2-n-propyl-3-[(2′-(tetrazol-5-yl)biphen-4-yl)methyl]pyrazino [3,2-d]pyrimidin-4(3H)-one;
(22) 6-(N-Benzoyl-N-n-pentyl)amino-2-n-propyl-3-[(2′-(tetrazol-5-yl)biphen-4-yl)methyl]pyrazino [3,2-d] pyrimidin-4(3H)-one;
(23) 6-(N-(p-Chloro)benzoyl-N-n-pentyl)amino-2-n-propyl-3-[(2′-(tetrazol-5-yl)biphen-4-yl)methyl]pyrazino[3,2-d]pyrimidin-4(3H)-one;
(24) 6-(N-(p-Chloro)benzoyl-N-isobutyl)amino-2-n-propyl-3-[(2′-(tetrazol-5-yl)biphen-4-yl)methyl] pyrazino[3,2-d]pyrimidin-4(3H)-one;
(25) 6-(N-n-Propyl-N-isobutyloxycarbonyl)amino-2-n-propyl-3-[(2′-(tetrazol-5-yl)biphen-4-yl)methyl] pyrazino[3,2-d]pyrimidin-4(3H)-one;
(26) 6-(N-Benzoyl-N-n-pentyl)amino-3-[(2′-(N-benzoylsulfonamido)biphen-4-yl)methyl]-2-n-propyl-pyrazino[3,2-d]pyrimidin-4(3H)-one;
(27) 2-n-Butyl-6-(N-methyl-N-isobutyloxycarbonyl) amino-3-[(2′-(tetrazol-5-yl)biphen-4-yl)methyl] pyrazino[2,3-d]pyrimidin-4(3H)-one;
(28) 6-(N-Benzyl-N-butanoyl)amino-2-n-propyl-3-[(2′-(tetrazol-5-yl)biphen-4-yl)methyl]pyrazino [2,3-d]pyrimidin-4(3H)-one;
(29) 6-(N-(p-Chloro)benzoyl-N-n-pentyl)amino-2-n-propyl-3-[(2′-(tetrazol-5-yl)biphen-4-yl)methyl]pyrazino[2,3-d]pyrimidin-4(3H)-one;
(30) 6-(N-n-Propyl-N-isobutyloxycarbonyl)amino-2-n-propyl-3-[(2′-(tetrazol-5-yl)biphen-4-yl)methyl] pyrazino[2,3-d]pyrimidin-4(3H)-one;
(31) 6-(N-Benzoyl-N-n-pentyl)amino-3-[(2′-(N-benzoylsulfonamido)biphen-4-yl)methyl]-2-n-propyl-pyrazino[2,3-d]pyrimidin-4(3H)-one,
(32) 2-N-butyl-3-[(2′-(N-cyclopropylcarbonylsulfonamido)biphen-4-yl)methyl]-6-(N-cyclopropylcarbonylpiperazin-1-yl)-pyrazino-[2,3-d]-pyrimidin-4(3H)-one.
(33) 6-(N-acetyl-piperazin-1-yl)-2-n-propyl-3-[(2′-(tetrazol-5-yl)biphen-4-yl)methyl]-pyrazino[2,3-d] pyrimidin-4(3H)-one.
(34) 6-(N-benzyl-N-benzoyl)-2-methyl-3-[(2′-(tetrazol-5-yl)biphen-4-yl)methyl]-pyrazino[2,3-d]-pyrimidin-4(3H)-one;
(35) 6-(n-benzyl-N-benzoyl)-2-isopropyl-3-[(2′-(tetrazol-5-yl)biphen-4-yl)methyl]-pyrazino-[2,3-d]pyrimidin-4(3H)-one, and
(36) 2-ethyl-6-(N-(2-thienylcarbonyl)-N-benzyl)3-[(2′-(tetrazol-5-yl)biphen-4-yl)methyl]pyrazino[2,3-d] pyrimidin-4(3H)-one.

**6.** The compound of Claim 4 which is:
6-Aminopentyl-2-propyl-3-[(2′-tetrazol-5-yl)biphen-4-yl)methyl]pyrazino[2,3-d]pyrimidin-4-(3H)-one;
6-Bromo-2-propyl-3-[(2′-tetrazol-5-yl)biphen-4-yl)methyl]pyrazino[2,3-d]pyrimidin-4(3H)-one; or
6-Morpholino-2-propyl-3-[(2′-tetrazol-5-yl)biphen-4-yl)methyl]pyrazino[2,3-d]pyrimidin-4-(3H)-one.

**7.** A pharmaceutical composition useful in the treatment of hypertension which comprises a pharmaceutically acceptable carrier and a therapeutically effective amount of a compound of Claim 1.

**8.** The composition of Claim 7 which includes another antihypertensive agent selected from a diuretic, an angiotensin converting enzyme inhibitor a calcium channel blocker and a β-blocker which are members selected from the group consisting of:
amiloride, atenolol, bendroflumethiazide, chlorothalidone, chlorothiazide, clonidine, cryptenamine acetates and cryptenamine tannates, deserpidine, diazoxide, guanethidine sulfate, hydralazine hydrochloride, hydrochlorothiazide, methyldopa, methyldopate hydrochloride, minoxidil, pargyline hydrochloride, polythiazide, prazosin, propranolol, rauwolfia serpentina, rescinnamine, reserpine, sodium nitroprusside, pironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflunisal, diltiazem, felodipine, nicardipine, nifedipine, niludipine, nimodipine, nisoldipine, nitrendipine, as well as admixtures and combinations thereof.

**9.** The use of a compound as claimed in claim 1 for the manufacture of a medicament for treating hypertension.

**10.** The use of a compound as claimed in claim 1 for the manufacture of a medicament for the treatment of ocular hypertension.